(19) Europäisches Patentamt / European Patent Office / Office européen des brevets

(11) **EP 3 708 076 A1**

(12) **EUROPEAN PATENT APPLICATION**
published in accordance with Art. 153(4) EPC

(43) Date of publication:
**16.09.2020 Bulletin 2020/38**

(21) Application number: **18875170.5**

(22) Date of filing: **23.10.2018**

(51) Int Cl.:
***A61B 5/053*** (2006.01)

(86) International application number:
**PCT/JP2018/039292**

(87) International publication number:
**WO 2019/093115 (16.05.2019 Gazette 2019/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.11.2017 JP 2017215847**

(71) Applicant: **Tanita Corporation**
**Tokyo 174-8630 (JP)**

(72) Inventors:
• **NAGAHAMA, Toshiki**
 **Tokyo 174-8630 (JP)**
• **SHIMIZU, Tomoka**
 **Tokyo 174-8630 (JP)**
• **KASAHARA, Yasuhiro**
 **Tokyo 174-8630 (JP)**

(74) Representative: **Grünecker Patent- und Rechtsanwälte**
**PartG mbB**
**Leopoldstraße 4**
**80802 München (DE)**

(54) **BODY COMPOSITION SCALE AND BODY COMPOSITION MEASUREMENT PROGRAM**

(57)    A body composition meter comprises: a multi-frequency current application unit to apply an AC current of multiple frequencies to a body of a subject; an impedance measurement unit to measure bioelectrical impedances of the subject when the AC current is applied by the multi-frequency current application unit at the respective frequencies; a body composition measurement unit to obtain parameters on a body composition of the subject based on the bioelectrical impedances measured by the impedance measurement unit; a measured data Cole circle calculation unit to calculate a Cole circle of measured data using resistances and reactances in the bioelectrical impedances at the respective frequencies as measured by the impedance measurement unit; and a measurement anomaly determination unit to determine the presence or absence of an anomaly in the measurement of the bioelectrical impedance based on an amount of shift of the Cole circle (CI) of the measured data calculated by the measured data Cole circle measurement unit relative to a Cole circle (C0) of normal data using normal data of bioelectrical impedance.

FIG. 2

Legend:
★ CENTER O₀ OF COLE CIRCLE C0 OF NORMAL DATA
— COLE CIRCLE C0 OF NORMAL DATA
○ MEASUREMENT POINT OF MEASURED DATA 1 AT EACH FREQUENCY
---- COLE CIRCLE C1 OF MEASURED DATA 1
☆ CENTER O₁ OF COLE CIRCLE C1 OF MEASURED DATA 1
● MEASUREMENT POINT OF MEASURED DATA 2 AT EACH FREQUENCY
—·— COLE CIRCLE C2 OF MEASURED DATA 2
✱ CENTER O₂ OF COLE CIRCLE C2 OF MEASURED DATA 2
— NORMAL RANGE LMT OF CENTER OF CIRCLE

EP 3 708 076 A1

**Description**

**TECHNICAL FIELD**

The present invention relates to a body composition meter and a body composition measurement program for measuring the body composition of a subject, and more specifically to a technology for determining an anomaly in the measurement of bioelectrical impedance used to measure a body composition.

**BACKGROUND ART**

Conventionally, there is known a body composition meter for measuring parameters on the body composition of a subject, such as body fat rate, visceral fat level and amount of muscle. This body composition meter measures the weight and bioelectrical impedance of a subject, and calculates parameters on the body composition of the subject such as body fat rate, visceral fat level and amount of muscle based on input information of the subject such as the gender, height and age in addition to the measured weight and bioelectrical impedance. Note that in the present specification, to measure (calculate) the parameters on the body composition such as body fat rate described above is referred to as to "measure the body composition".

In the field of this kind of device, a bioimpedance measurement device is known which detects an anomaly in the measurement of bio(electrical) impedance when the contact impedance (contact resistance) between the body (precisely, skin of a hand or foot) of a subject and measurement electrodes (current application electrode and voltage measurement electrode) is high due to, for example, smallness of a contact area between the body of the subject and the measurement electrodes (refer to Patent Document 1). This device comprises a constant current source to allow an AC current of a constant value to flow in the body of the subject, and detects (determines) that an anomaly occurs in the measurement of bioimpedance because the contact impedance (contact resistance) between the body of the subject and the measurement electrodes is high when the supply voltage from the constant current source to the current application electrode exceeds an allowable supply voltage range set within the range of maximum supply voltage, or when the input voltage from the voltage measurement electrode to a differential amplifier circuitry exceeds the allowable supply voltage range set within the range of maximum supply voltage. Note that the contact impedance described above refers to impedance generated between the measurement electrode and the body of the subject.

Also in the field of this kind of device, a body composition estimation device is known which is configured to reduce incorrect determination of the body composition of a subject due to the output of an inaccurately estimated value of the body composition, for example, when the posture of the subject is not desirable, or when a part of the body of the subject contacts a metal material such as a bed frame (refer to Patent Document 2). This device calculates an impedance locus based on measured values of the bioelectrical impedance of the subject, and determines that an anomaly has occurred in the measurement of the bioelectrical impedance, based on the magnitude of variation of the measured values of the bioelectrical impedance of the subject at respective frequencies relative to the impedance locus. Specifically, this device determines that an anomaly has occurred in the measurement of the bioelectrical impedance, when $\sum e(f)^2$, or $\sum e'(f)^2$ calculated by the following equation (1), or $\sum e''(f)^2$ calculated by the following equation (2) exceeds a predetermined threshold:

$$\sum e'(f)^2 = \sum \{e(f)/r\}^2 \qquad (1)$$

$$\sum e''(f)^2 = \sum \{e(f)/h\}^2 \qquad (2)$$

Here, e(f) represents distance in a given direction between a bioelectrical impedance at frequency f and the impedance locus calculated from the measured bioelectrical impedances at the respective frequencies, while r represents radius of the impedance locus, and h represents height of the subject.

**PRIOR ART DOCUMENTS**

**PATENT DOCUMENTS**

[Patent Document 1] Japanese Laid-open Patent Publication 2012-213458

[Patent Document 2] Japanese Patent 5110277

## SUMMARY OF THE INVENTION

### PROBLEM TO BE SOLVED BY THE INVENTION

Generally, main situations (causes) to bring about an anomaly in the measured values of bioelectrical impedance of a body composition meter can be given as follows:

- The case where a hand or a foot of a subject does not firmly contact the electrode;
- The case where the hand or the foot of the subject is extremely dry;
- The case where the skin of the palm or the foot sole of the subject is extremely thick;
- The case where there is a contact between parts of the body of the subject with each other, such as between an upper limb and the body trunk, or between the left and right lower limbs with each other (the case where electric conduction occurs between parts of the body of the subject with each other due to a contact between skins of the subject with each other); and
- The case where the body of the subjects contacts an electrically conductive material such as metal.

Among the situations described above, the device described in Patent Document 1 above can detect an anomaly in the measurement of bioelectrical impedance which occurs when the contact impedance (contact resistance) between the body of the subject and the measurement electrode is high, such as when the hand or the foot of the subject does not contact the electrode, or when the hand or the foot is extremely dry. However, for example, when parts of the body of the subject, such as an upper limb and the body trunk, contact with each other, the contact resistance between the body of the subject and the measurement electrode is not necessarily higher than when there is no contact between parts of the body of the subject with each other. Therefore, the device described in Patent Document 1 above cannot detect an anomaly in the measurement of bioelectrical impedance due to a contact between parts of the body of the subject with each other.

Further, the device described in Patent Document 2 above calculates an impedance locus based on measured values of the bioelectrical impedance of the subject, and determines an anomaly in the measurement of the bioelectrical impedance due to disturbance such as contact failure of the electrode, based on the magnitude of variation of the measured values of the bioelectrical impedance of the subject at respective frequencies relative to the impedance locus. However, among the situations to cause the anomaly in the measurement described above, when parts of the body of the subject contact with each other (including when the parts of the body indirectly contact with each other through an electrically conductive material such as metal), the variation of the measured values of the bioelectrical impedance at the respective frequencies relative to the impedance locus is not necessarily large. Therefore, there is a possibility that the method to determine an anomaly in the measurement of bioelectrical impedance based on the magnitude of this variation may not be able to accurately detect an anomaly in the measurement of bioelectrical impedance.

An object of the present invention is to solve the problems described above and to provide a body composition meter and a body composition measurement program that can accurately detect an anomaly in the measurement of bioelectrical impedance due to a contact between parts of a subject with each other

### MEANS TO SOLVE THE PROBLEM

In order to solve the above problem, a body composition meter according to a first aspect of the present invention comprises: a multi-frequency current application unit configured to apply an AC current of multiple frequencies to a body of a subject; an impedance measurement unit configured to measure bioelectrical impedances of the subject when the AC current is applied by the multi-frequency current application unit at the respective frequencies; a body composition measurement unit configured to obtain parameters on a body composition of the subject based on the bioelectrical impedances measured by the impedance measurement unit; a measured data Cole circle calculation unit configured to calculate a Cole circle of measured data using resistances and reactances in the bioelectrical impedances at the respective frequencies as measured by the impedance measurement unit; and a measurement anomaly determination unit configured to determine the presence or absence of an anomaly in the measurement of the bioelectrical impedance based on an amount of shift of the Cole circle of the measured data calculated by the measured data Cole circle calculation unit relative to a Cole circle of normal data using normal data of bioelectrical impedance. Note that in the present specification, the "Cole circle" means a circle according to the so-called Cole-Cole circular arc law. Further, in the present specification, the "Cole-Cole circular arc law" means a law that when resistive (resistance) components and capacitive components (negative reactance components) of impedances collected at various frequencies are plotted on a complex plane, the locus of the plotted points has a circular arc shape such as a half circle.

A body composition measurement program according to a second aspect of the present invention causes a computer to execute the steps of: measuring bioelectrical impedances of a subject when an AC current of multiple frequencies is applied to a body of the subject at the respective frequencies; obtaining parameters on a body composition of the subject based on the bioelectrical impedances measured by the step of measuring the bioelectrical impedance of the subject; calculating a Cole circle of measured data using resistances and reactances in the bioelectrical impedances at the respective frequencies as measured by the step of measuring the bioelectrical impedance of the subject; and determining the presence or absence of an anomaly in the measurement of the bioelectrical impedance based on an amount of shift of the Cole circle of the measured data calculated by the step of calculating the Cole circle of the measured data relative to a Cole circle of normal data using normal data of bioelectrical impedance.

## EFFECTS OF THE INVENTION

The body composition meter or the body composition measurement program of the present invention determine the presence or absence of an anomaly in the measurement of bioelectrical impedance based on an amount of shift of a Cole circle of measured data relative to a Cole circle of normal data calculated using data of normal bioelectrical impedance. Thus, in contrast to the device described in Patent Document 2 above, the presence or absence of an anomaly in the measurement of bioelectrical impedance can be accurately determined by using the Cole circle of normal data as a reference, even if it is difficult to determine the anomaly in the measurement of bioelectrical impedance based on the magnitude of variation of measured values of the bioelectrical impedance of a subject at the respective frequencies relative to an impedance locus, as in the case where parts of the body of the subject contact with each other. Therefore, it is possible to accurately detect an anomaly in the measurement of bioelectrical impedance due to a contact between parts of the body of the subject with each other.

## BRIEF DESCRIPTION OF THE DRAWINGS

[FIG. 1] Schematic electric block diagram of a body composition meter according to a first exemplary embodiment of the present invention.
[FIG. 2] Explanatory view of a normal range of the center of a Cole circle obtained from normal data of a certain part of a subject along with the centers of Cole circles obtained from measured data at the time of anomaly in the measurement in the body composition meter.
[FIG. 3] (a) and (b) are, respectively, an explanatory view showing a drawing method of a circle passing through three points A, B, C, and an explanatory view showing a drawing method of a circle passing through these two points A, B in the case where the value on the x-axis of one point A among the points A, B is equal to the value on the x-axis of the center $O_a$ of a circle.
[FIG. 4] View showing a comparison of a normal range of bioelectrical impedance at a certain frequency as calculated from group normal data with a normal range of bioelectrical impedance at the same frequency as calculated from intra-individual normal data.
[FIG. 5] View showing an electrical equivalent circuitry of a biological tissue.
[FIG. 6] View showing an electrical equivalent circuitry of upper limbs of a human body.
[FIG. 7] View showing an electrical equivalent circuitry when an upper limb contacts with the body trunk.
[FIG. 8] View in which measured values of resistance and reactance of a right arm collected by applying currents of respective frequencies at normal time and at the time of contact in the body composition meter are plotted on a complex plane.
[FIG. 9] View showing a locus of the Cole-Cole plot at measurement points as generated based on the measured data of the right arm at the normal time and the measured data of the right arm at the time of contact in contact with the right side.
[FIG. 10] Graph showing time series data of contact impedance in the body composition meter when the hand and the foot of the subject are dry.
[FIG. 11] Flow chart of processes for determination of an anomaly in the measurement of bioelectrical impedance in the body composition meter.
[FIG. 12] View showing measurement paths of bioelectrical impedance of respective parts in the body composition meter.
[FIG. 13] Explanatory view of a remeasurement process performed by changing the measurement path in the case where it is difficult to contact an electrode with one hand or one foot in the body composition meter.
[FIG. 14] Schematic electric block diagram of a body composition meter according to a second exemplary embodiment of the present invention.

[FIG. 15] Explanatory view of a correction method of measured data using a Cole circle according to Modified Example 1 of the present invention.

**DESCRIPTION OF EMBODIMENTS**

Hereinafter, a body composition meter according to exemplary embodiments of the present invention will be described with reference to the drawings.

<1. Configuration of body composition meter>

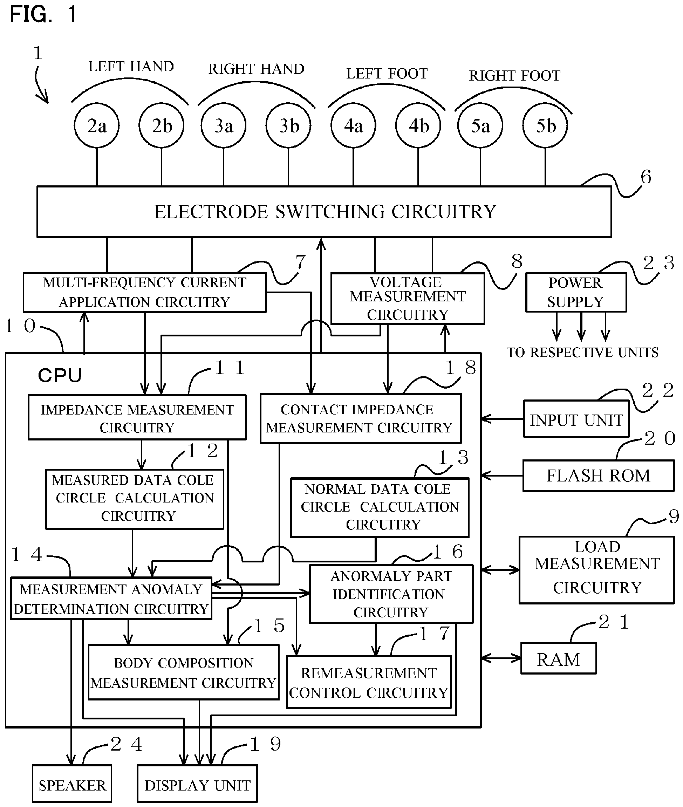

FIG. 1 shows an electric block diagram of a body composition meter according to a first exemplary embodiment of the present invention. The body composition meter 1 comprises current application electrodes 2a, 3a and voltage measurement electrodes 2b, 3b to be contacted with the left and right palms of a subject as well as current application electrodes 4a, 5a and voltage measurement electrodes 4b, 5b to be contacted with the left and right fool soles of the subject when measuring the bioelectrical impedance of the subject. In other words, the body composition meter 1 is an 8-electrode type body composition meter to contact eight measurement electrodes in total (current application electrodes 2a, 3a, 4a, 5a and voltage measurement electrodes 2b, 3b, 4b, 5b) with both hands and both feet to measure the bioelectrical impedance of the subject. Note that in the present specification, the current application electrodes 2a, 3a, 4a, 5a and the voltage measurement electrodes 2b, 3b, 4b, 5b may be referred to collectively as hand/foot electrodes.

Further, the body composition meter 1 comprises an electrode switching circuitry 6, a multi-frequency current application circuitry 7, a voltage measurement circuitry 8, a load measurement circuitry 9, a CPU 10, a display unit 19, a flash ROM (Read Only Memory) 20, a RAM (Random Access Memory) 21, an input unit 22, a power supply 23 and a speaker 24.

The electrode switching circuitry 6 is a circuitry (change-over switch) configured to switch the electrodes (switch impedance measurement paths) used to measure the bioelectrical impedance. Based on a control signal from the CPU 10, the electrode switching circuitry 6 selectively connects two electrodes out of the current application electrodes 2a, 3a, 4a, 5a to the multi-frequency current application circuitry 7, and also selectively connects two electrodes out of the voltage measurement electrodes 2b, 3b, 4b, 5b to the voltage measurement circuitry 8.

The multi-frequency current application circuitry 7 (corresponding to the "multi-frequency current application unit" in the claims) is a current source which can apply AC currents of multiple frequencies, and applies a current of a frequency commanded by the CPU 10 between two current application electrodes connected through the electrode switching circuitry 6. Further, the multi-frequency current application circuitry 7 has a built-in output current detection circuitry (not shown), and uses the output current detection circuitry to detect a current value of the supplied AC current, and also outputs the detected current value to an impedance measurement circuitry 11 of the CPU 10.

When current is applied from the above-described multi-frequency current application circuitry 7, the voltage measurement circuitry 8 detects (measures) a voltage corresponding to a potential difference between the two voltage measurement electrodes connected through the electrode switching circuitry 6 (that is, potential difference generated between selected parts of the body of the subject).

The load measurement circuitry 9 measures the weight of the subject placed on an upper surface of a housing of the body composition meter 1. The load measurement circuitry 9 has a load cell to convert a load to an electric signal (output an electric signal of a value corresponding to the load). As the load cell, the present embodiment uses a so-called strain gauge type (a type that has a strain material which deforms according to load when the load is applied thereto, and that also has a strain gauge attached to the strain material to output a voltage of a value corresponding to a change in the resistance value of the strain gauge due to the load). When the subject is placed on the upper surface of the housing of the body composition meter 1, the (load cell of the) load measurement circuitry 9 changes in its output value in response to the weight of the subject (voltage value corresponding to a change in the resistance value of the strain gauge due to the weight of the subject). The output value from the load measurement circuitry 9 is converted by an A/D converter (not shown) to a digital value, and then sent as weight data to the CPU 10. Based on the weight data, the CPU 10 calculates the weight of the subject according to a program stored in the flash ROM 20, and stores the calculated weight in the RAM 21.

The CPU 10 controls the entire device of the body composition meter 1 and performs various operations. As shown in FIG. 1, the CPU 10 comprises the impedance measurement circuitry 11, a measured data Cole circle calculation circuitry 12, a normal data Cole circle calculation circuitry 13, a measurement anomaly determination circuitry 14, a body composition measurement circuitry 15, an anomaly part identification circuitry 16, a remeasurement control circuitry 17 and a contact impedance measurement circuitry 18. Note that the CPU 10 including these circuitries 11 to 18 and the voltage measurement circuitry 8 described above are circuitries formed by basic function blocks of a microcomputer. The impedance measurement circuitry 11, the measured data Cole circle calculation circuitry 12, the measurement anomaly determination circuitry 14, the body composition measurement circuitry 15, the anomaly part identification circuitry 16, the remeasurement control circuitry 17 and the contact impedance measurement circuitry 18 correspond to the impedance measurement unit, the measured data Cole circle calculation unit, the measurement anomaly determination unit, the body composition measurement unit, the anomaly part identification unit, the remeasurement control unit and the contact impedance measurement unit, respectively, in the claims.

Further, the CPU 10 reads programs stored in the flash ROM 20 so as to cause the body composition meter 1, which is a computer, to execute the respective steps for the determination of an anomaly in the impedance measurement as will be described below.

The impedance measurement circuitry 11 measures a bioelectrical impedance Z of a subject, when an AC current of each frequency is applied by the multi-frequency current application circuitry 7, based on a current value of the AC current detected by the output current detection circuitry in the multi-frequency current application circuitry 7 and a voltage value detected by the voltage measurement circuitry 8. Specifically, first, the impedance measurement circuitry 11 calculates an absolute value $|Z|$ of the bioelectrical impedance based on the current value of the AC current detected by the output current detection circuitry in the multi-frequency current application circuitry 7 and the voltage value detected by the voltage measurement circuitry 8. Further, based on a current waveform obtained using the current value of the AC current detected by the output current detection circuitry and on a voltage waveform obtained using the voltage value detected by the voltage detection circuitry 8, the impedance measurement circuitry 11 obtains a phase angle (impedance angle) $\varphi$ which is an amount of phase shift of the voltage relative to the current. Then, the impedance measurement circuitry 11 calculates a resistance R and a reactance X based on the absolute value $|Z|$ of the bioelectrical impedance and the phase angle $\varphi$. Note that it is also possible to obtain the phase angle $\varphi$ by using a voltage waveform measured based on the voltage value detected by the voltage measurement circuitry 8 and by using a voltage waveform generated when a current, which is the same (in frequency and magnitude) as the AC current used to measure the voltage waveform, flows through a reference resistance.

The measured data Cole circle calculation circuitry 12 uses the resistance R and the reactance X in the bioelectrical impedance Z (of a part to be measured) at respective frequencies as measured by the impedance measurement circuitry 11 to calculate a Cole circle of the measured data. In the present specification, the "Cole circle" means a circle according to the so-called Cole-Cole circular arc law. Note that in the present specification, the "Cole-Cole circular arc law" means a law that when measured values (resistive (resistance) components and capacitive components (negative reactance components) of impedances) collected at various frequencies are plotted on a complex plane (are subjected to the "Cole-Cole plotting"), the locus of the plotted points has a circular arc shape such as a half circle.

The normal data Cole circle calculation circuitry 13 calculates a Cole circle of normal data by using resistances R' and reactances X' in the data of normal bioelectrical impedances Z' at the respective frequencies of the part to be measured which are stored in the flash ROM 20. The data of the normal bioelectrical impedances Z' can be data of past normal bioelectrical impedances (when there was no anomaly in measurement) of the subject itself to be measured currently, or average data of normal bioelectrical impedances collected from a group of multiple people.

The measurement anomaly determination circuitry 14 determines the presence or absence of an anomaly in the measurement of the bioelectrical impedance Z based on an amount of shift of the Cole circle of the measured data calculated by the measured data Cole circle calculation circuitry 12 relative to the Cole circle of the normal data calculated by the normal data Cole circle calculation circuitry 13.

The body composition measurement circuitry 15 obtains parameters on the body composition of the subject based on the bioelectrical impedance Z (resistance R and reactance X) measured by the impedance measurement circuitry 11. More specifically, the body composition measurement circuitry 15 calculates parameters on the body composition of the subject such as body fate rate, amount of muscle, estimated amount of bone, visceral fat level, amount of basal metabolism and body water rate, based on the bioelectrical impedance Z (resistance R and reactance X) measured by the impedance measurement circuitry 11, the weight of the subject stored in the RAM 21 and personal data such as height, age and gender of the subject input by the subject (user) using the input unit 22.

When the measurement anomaly determination circuitry 14 determines the present or absence of an anomaly in the measurement of the bioelectrical impedance Z along a plurality of impedance measurement paths (hereafter referred to as "measurement paths") in the body of the subject, and determines the presence of an anomaly in one of the plurality of measurement paths, the anomaly part identification circuitry 16 identifies a part of the body of the subject acting as a source of producing an anomaly in the measurement, based on a result of the determination of anomaly in the measurement with respect to the plurality of measurement paths. When an anomaly in measurement is present in one of the plurality of measurement paths, the remeasurement control circuitry 17 controls the impedance measurement circuitry 11 to remeasure the bioelectrical impedance Z of the part acting as the source of producing the anomaly in the measurement by using a measurement path different from the measurement path which is normally used for the impedance measurement of this part. The contact impedance measurement circuitry 18 measures a contact impedance generated between a measurement electrode used to measure the bioelectrical impedance of the subject and the body (mainly, a hand or a foot) of the subject. Here, the measurement path includes a path (current path) between current application electrodes and a path (voltage measurement path) between voltage measurement electrodes.

The display unit 19 displays various information such as operation guide information for users (subjects), results of determinations of anomaly in measurement by the measurement anomaly determination circuitry 14, and parameters on the body composition measured by the body composition measurement circuitry 15. In the present exemplary embodiment, the display unit 19 is formed of a liquid crystal display provided with a back light, but can be formed of another kind of display unit such as an organic EL display. The speaker 24 outputs guide voice for users (subjects), anomaly notification sound, and so on.

The flash ROM 20 is a rewritable non-volatile memory, and stores various programs, set data, personal data of a subject such as height, age and gender, historical data of measured values of the subject, and so on. Further, the flash ROM 20 stores a normal database. This normal database stores data of the normal bioelectrical impedances Z' of each part to be measured at the respective frequencies as described above. The RAM 21 is used as a working area of the CPU 10 when various programs are executed. The input unit 22 is used for various input operations including command input to the body composition meter 1, and input of personal data of the subject such as height, age and gender required for measurement, and so on. The power supply 23 supplies power to respective units of the body composition meter 1.

<2. Determination of anomaly in measurement of impedance>

<2-1. Kind of anomaly in measurement of impedance>

Concerning the bioelectrical impedance Z of a subject measured by the impedance measurement circuitry 11, a situation may occur that, for various reasons, an impedance Z with a value different from the impedance Z which should normally be measured is measured. This situation can be referred to as an anomaly in the measurement of impedance. It is considered that the main causes to produce an anomaly in the measurement of impedance are: (1) parts of the body of the subject contact with each other; (2) the hand or the foot of the subject does not firmly contact with the electrode (contact failure between the hand/foot electrodes and the body of the subject); and (3) the hand or the foot of the subject is extremely dry. The body composition meter according to the present invention determines the occurrence of an anomaly in the measurement of impedance due to (1) at least.

<2-2. Determination of anomaly in measurement of impedance due to cause (1)>

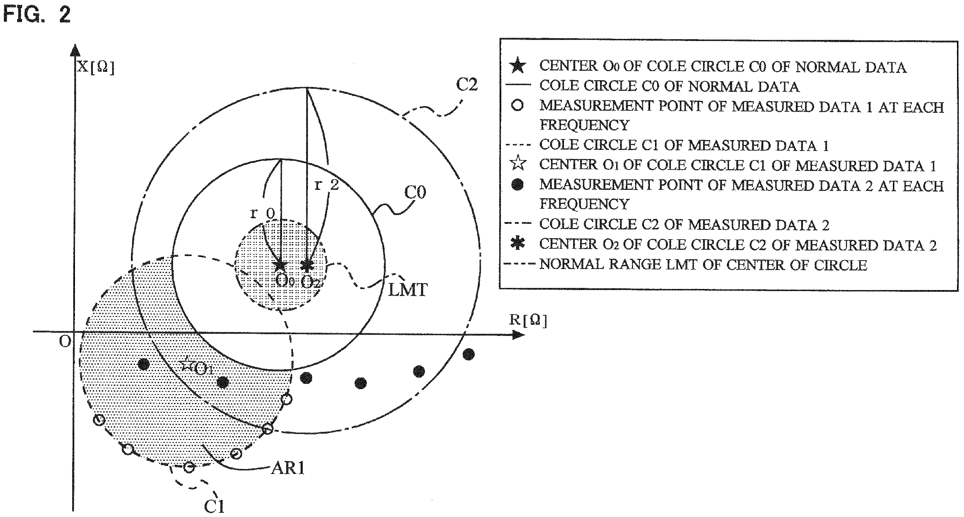

Next, referring to FIG. 2, an outline of a determination process of an anomaly in the measurement of bioelectrical impedance in the body composition meter 1 of the present exemplary embodiment due to a contact between parts of the body of a subject with each other will be described. The present body composition meter 1 determines the presence or absence of an anomaly in the measurement of the bioelectrical impedance Z mainly based on an amount of shift of the Cole circle of the measured data calculated by the measured data Cole circle calculation circuitry 12 relative to the Cole circle of the normal data calculated by the normal data Cole circle calculation circuitry 13. As described above, the normal data Cole circle calculation circuitry 13 calculates a Cole circle C0 of the normal data in FIG. 2 by using the resistances R' and the reactances X' in the data of the normal bioelectrical impedances Z' at the respective frequencies of a part to be measured which are stored in the normal database. Further, the measured data Cole circle calculation circuitry 12 calculates a Cole circle of the measured data by using the resistances R and the reactances X at the respective frequencies of a part to be measured which are measured by the impedance measurement circuitry 11. As examples of the Cole circle of measured data, FIG. 2 shows a Cole circle C1 of measured data 1, and a Cole circle C2 of measured data 2 which is a measured data different from the measured data 1. Note that in FIG. 2, the circular arcs of the Cole circles C0, C1 and C2 are also drawn in the positive region of the reactance X on the premise that the Cole circles are circles according to the so-called Cole-Cole circular arc law. However, since the biological body does not include a coil component, actual measured values are not present in the positive region of the reactance X.

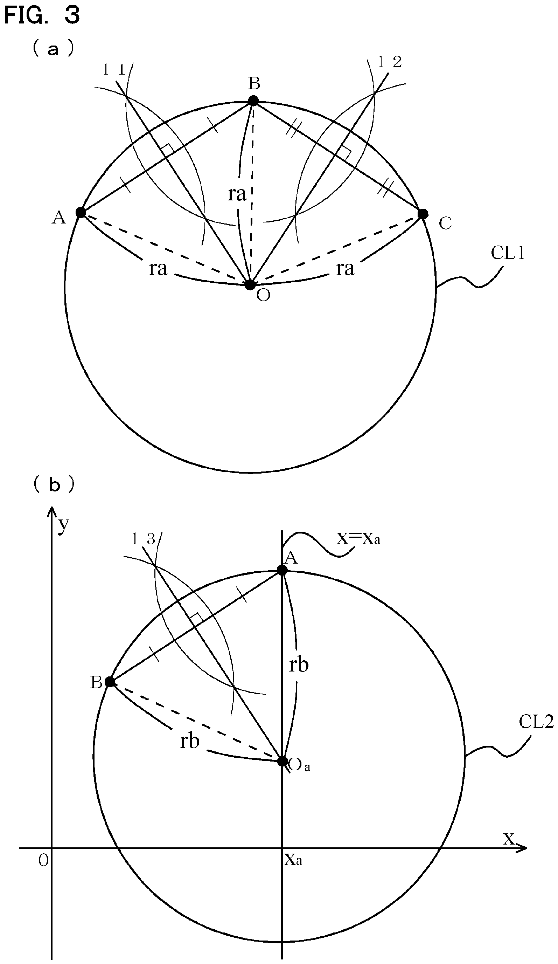

Generally, a Cole circle can be obtained from points (at least three measurement points) on a complex plane corresponding to the resistances and reactances measured at three or more frequencies. This is because generally, as shown in FIG. 3(a), a circle CL1 passing through three points A, B, C is uniquely determined. FIG. 3(a) shows a drawing method of a circle passing through three points A, B, C. As shown in this Figure, the center O of the circle CL1 passing through the three points A, B, C is an intersection point between a perpendicular bisector 11 of chord AB and a perpendicular bisector 12 of chord BC, while the radius of the circle CL1 ra=OA=OB=OC. Note that when obtaining a Cole circle from four measurement points or more, the least squares method is used to obtain a circle which minimizes the difference (amount of shift) between the locus of the circle and four or more measurement points plotted on a complex plane.

Further, using the knowledge that in the measurement of bioelectrical impedance, the value of a resistance R measured at a frequency of at least 20 kHz and at most 100 kHz is approximate to the value on the x-axis of the center of a Cole circle on a complex plane, it is possible to obtain a Cole circle from points (measurement points) on a complex plane which correspond to resistances and reactances measured at two frequencies more including at least a frequency of at least 20 kHz and at most 100 kHz. Note that if a plurality of measurement results at frequencies of at least 20 kHz and at most 100 kHz are included, the value of resistance R according to a measurement result at a frequency which minimizes the reactance is used as the value on the x-axis of the center of the Cole circle on the complex plane. This is because generally, as shown in FIG. 3(b), when the value on the x-axis of one of two points A, B is equal to the value $X_a$ on the x-axis of the center $O_a$ of a circle, a circle CL2 passing through these two points A, B is uniquely determined generally. FIG. 3(b) shows a drawing method of a circle passing through the two points A, B in the case where the value on the x-axis of one point A of these two points A, B is equal to the value $X_a$ on the x-axis of the center $O_a$ of the circle. As shown in this Figure, the center $O_a$ of the circle CL2 passing through the two points A, B is an intersection point between a perpendicular bisector 13 of chord AB and a line $X=X_a$ parallel to the y-axis, while the radius of the circle CL2 $rb=O_aA=O_aB$.

The present body composition meter 1 determines the presence or absence of an anomaly in the measurement of the bioelectrical impedance Z mainly based on an amount of shift of the Cole circle of the measured data calculated by the measured data Cole circle calculation circuitry 12 relative to the Cole circle C0 of the normal data calculated by the normal data Cole circle calculation circuitry 13. Specifically, for example, in a case such as the Cole circle C1 of the measured data 1 shown by the dashed line in FIG. 2 where the center $O_1$ of the Cole circle calculated from the measured data falls out of a normal range LMT (is outside the normal range LMT) of the center of circle calculated from the center $O_0$ of the Cole circle C0 of the normal data, the measurement anomaly determination circuitry 14 determines the presence of an anomaly in the measurement of bioelectrical impedance (that an anomaly in measurement occurs). In other words, the measurement anomaly determination circuitry 14 determines the presence or absence of an anomaly in the measurement of the bioelectrical impedance Z based on an amount of shift of the center $O_1$ of the Cole circle C1 of the measured data 1 relative to the center $O_0$ of the Cole circle $C_0$ of the normal data.

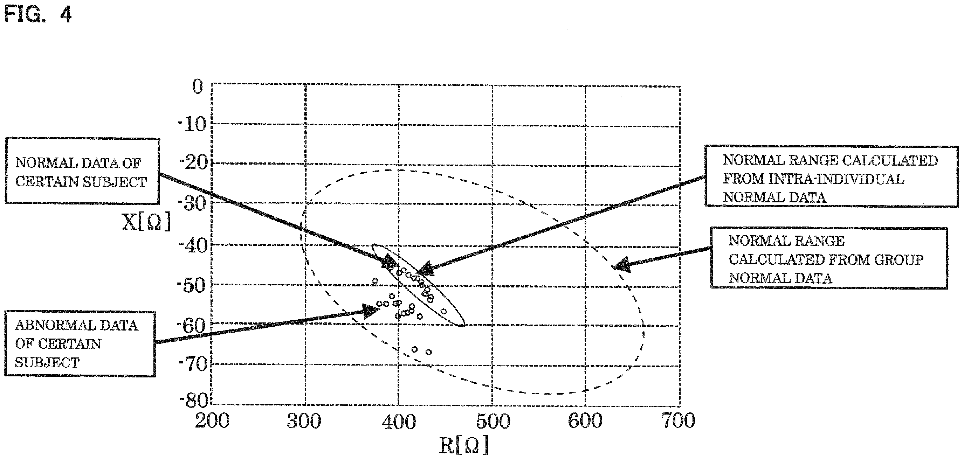

The normal database described above can be made from data of normal bioelectrical impedances Z' (group normal data) collected from a group of many normal subjects, or can be made from data of past normal bioelectrical impedances Z' (intra-individual normal data) of the subject itself to be measured currently. FIG. 4 shows a comparison of a normal range of the data of the bioelectrical impedance Z (data of resistance R and reactance X) at a certain frequency as produced (calculated) from the intra-individual normal data with a normal range of the data of the bioelectrical impedance Z at the same frequency as calculated from the group normal data. These normal ranges are shown by equal probability ellipses (confidence ellipses) with a given percentage (for example, 95%).

As shown in FIG. 4, the detection rate of an anomaly in the measurement of bioelectrical impedance is increased by producing (calculating) the normal range from the intra-individual normal data. Note that the "normal data of a certain subject" in FIG. 4 means the intra-individual normal data described above. Further, the "abnormal data of a certain subject" in FIG. 4 shows an example of data of the bioelectrical impedance Z (data of resistance R and reactance X) when an anomaly in the measurement of bioelectrical impedance occurs due to a contact between parts of the body of a certain subject with each other. However, also when an anomaly in the measurement of bioelectrical impedance occurs due to another cause, the "abnormal data of a certain subject" is highly likely to fall out of the "normal range calculated from intra-individual normal data" in FIG. 4.

The above example has described an example in which the "amount of shift of a Cole circle of measured data" (relative to a Cole circle of normal data) in the claims is an amount of shift of the center $O_1$ of the Cole circle C1 of the measured data 1 relative to the center $O_0$ of the Cole circle of the normal data. However, as shown in FIG. 2, the measurement anomaly determination circuitry 14 can also determine the presence or absence of an anomaly in the measurement of the bioelectrical impedance Z based on an area AR1 which is a region within the Cole circle C1 of the measured data 1 as calculated by the measured data Cole circle calculation circuitry 12, and which is excluded from within the Cole circle of the normal data as calculated by the normal data Cole circle calculation circuitry 13. In this case, the "amount of shift of a Cole circle of measured data" (relative to a Cole circle of normal data) in the claims is the area of AR1 which is a region within the Cole circle C1 of the measured data 1, and which is excluded from within the Cole circle of the normal data.

Further, as shown in FIG. 2, the measurement anomaly determination circuitry 14 can also determine the presence or absence of an anomaly in the measurement of bioelectrical impedance based on an amount of difference between a radius r0 of the Cole circle of the normal data and a radius r2 of the Cole circle C2 of the measured data 2 as calculated by the measured data Cole circle calculation circuitry 12. In this case, the "amount of shift of a Cole circle of measured data" (relative to a Cole circle of normal data) in the claims is an amount of difference (r2-r0) between the radius r0 of the Cole circle of the normal data and the radius r2 of the Cole circle C2 of the measured data 2.

Next, a process to determine the presence or absence of an anomaly in the measurement of bioelectrical impedance due to a contact between parts of the body of a subject with each other, as performed by the body composition meter 1 of the present exemplary embodiment, will be described. As described above, the measurement anomaly determination circuitry 14 can determine the presence or absence of an anomaly in the measurement of the bioelectrical impedance Z based only on an amount of shift of the center $O_1$ of the Cole circle C1 of the measured data 1 relative to

the center $O_0$ of the Cole circle of the normal data. However, in the case where the presence or absence of an anomaly in the measurement of the bioelectrical impedance Z due to a contact between parts of the body of a subject is determined, it is possible to more accurately determine the present or absence of the anomaly in the measurement by determining the presence or absence of the anomaly in the measurement based on a direction and a distance in the x-axis direction (a distance in the direction of x-axis) of the center of the Cole circle of the measured data relative to the center of the Cole circle of the normal data.

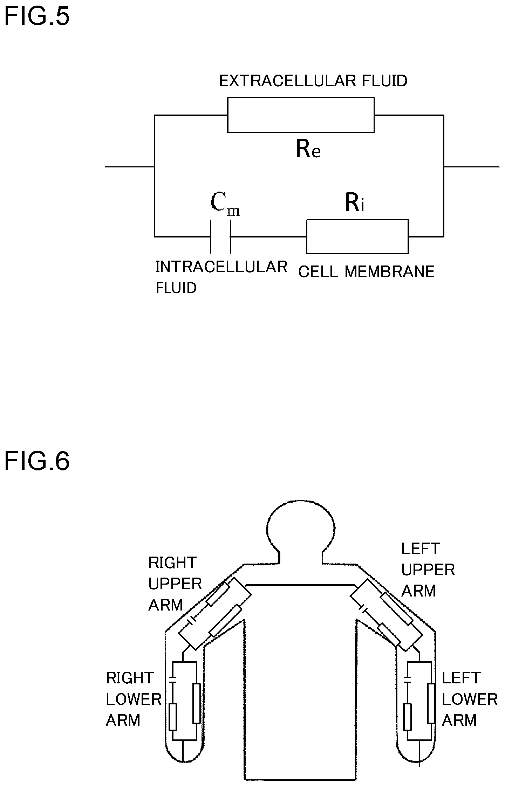

Next, referring to FIG. 5 to FIG. 9, the reasons why an anomaly in the measurement of the bioelectrical impedance Z due to a contact between parts of the body of a subject with each other can be accurately determined based on the direction, and the distance in the direction of the x-axis, of the center of the Cole circle of measured data will be described. Note that contacts which actually occur between parts of the body of a subject in the measurement of the bioelectrical impedance Z are mainly a contact between an upper limb and the body trunk of the subject, and a contact between the left and right lower limbs of the subject with each other. Further, an anomaly in the measurement of the bioelectrical impedance Z due to a contact between parts of the body of a subject may occur not only when parts of the body directly contact with each other, but also when they indirectly contact with each other through an electrically conductive material such as metal other than the electrodes. Note that in the following description, the posture of a subject when there is no contact between parts of the body of the subject with each other is defined as normal posture.

Generally, the electrical properties (resistance R and reactance X) of a biological tissue is represented by an electrical equivalent circuitry as shown in FIG. 5. More specifically, an extracellular fluid and an intracellular fluid are represented by resistive components (resistances) $R_e$, $R_i$, while a cell membrane is represented by a capacitive component (negative reactance) $C_m$. As shown in FIG. 6, an actual human body forms a series connection of a plurality of circuits one of which is shown in FIG. 5. Here, among the contacts between parts of the body of a subject with each other, the case of the contact between an upper limb (left/right arm) and the body trunk is used as an example for the description. However, this applies similarly to the case of the contact between left and right lower limbs, and the contact between an upper limb (left/right arm) and a left/right lower limb.

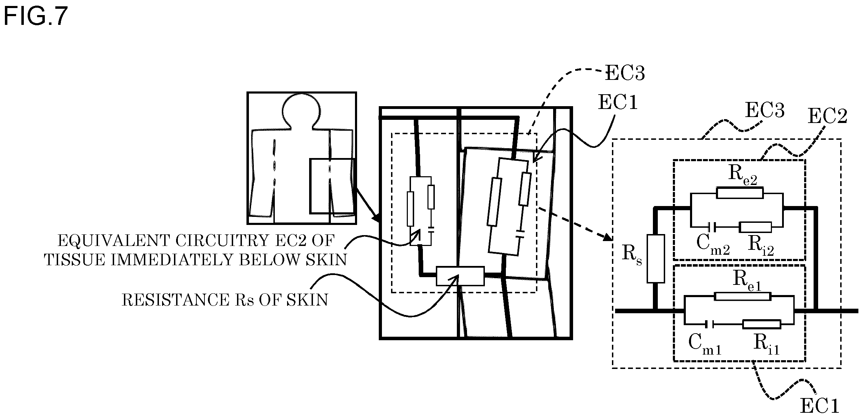

FIG. 7 shows an electrical equivalent circuitry EC3 formed by an upper limb (precisely, left upper arm) and a body trunk when the upper limb (left upper arm) contacts with the body trunk. When the upper limb contacts with the body trunk, as shown in FIG. 7, an electrical equivalent circuitry corresponding to tissue of the part connected with the upper limb (a resistance $R_s$ of skin and an electrical equivalent circuitry EC2 of tissue immediately below the skin) is added in parallel to an electrical equivalent circuitry EC1 of a left upper limb or a right upper limb (the left upper arm in FIG. 7). Therefore, as compared with measured values of the bioelectrical impedance Z in the normal posture, the resistances R and the reactances X significantly change, and fall out of the ranges of the resistance R and the reactance X when correct measurements are performed. Here, in FIG. 7, $R_{e1}$, $R_{i1}$, $C_{m1}$ represent extracellular fluid resistance, intracellular fluid resistance and cell membrane electrical capacitance of an upper limb (precisely, left upper arm), respectively, while $R_{e2}$, $R_{i2}$, $C_{m2}$ represent extracellular fluid resistance, intracellular fluid resistance and cell membrane electrical capacitance of tissue immediately below the skin, respectively, and $R_s$ represents contact resistance between the skins. The reason why the contact resistance $R_s$ between the skins does not include a capacitive component is because the skin has so little water as to make the electrical capacitance of the skin negligible. Note that FIG. 7 shows the electrical equivalent circuitry EC3 when there is a contact between the left upper arm and the body trunk among the contacts between an upper limb and the body trunk. However, this applies similarly to the electrical equivalent circuitry when a right upper arm, a left lower arm or a right lower arm contacts with the body trunk. Further, the electrical equivalent circuits, when left and right lower limbs contact with each other, and when an upper limb (left/right arm) contact with a left/right lower limb, are similar to the electrical equivalent circuitry EC3 shown in FIG. 7.

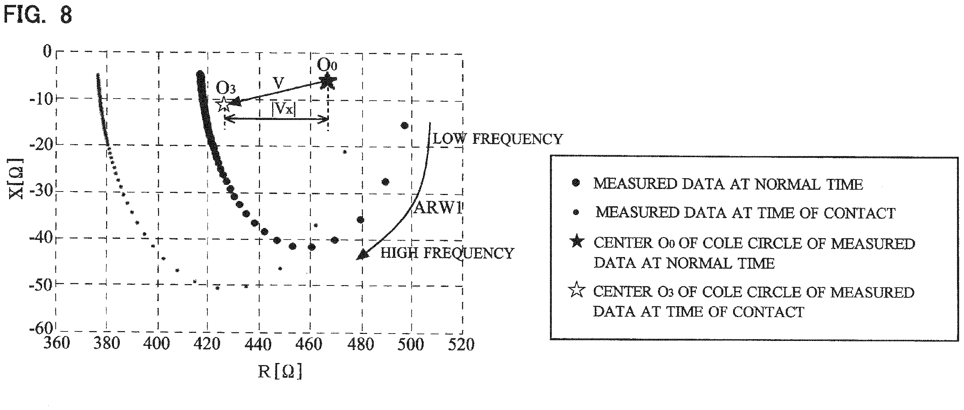

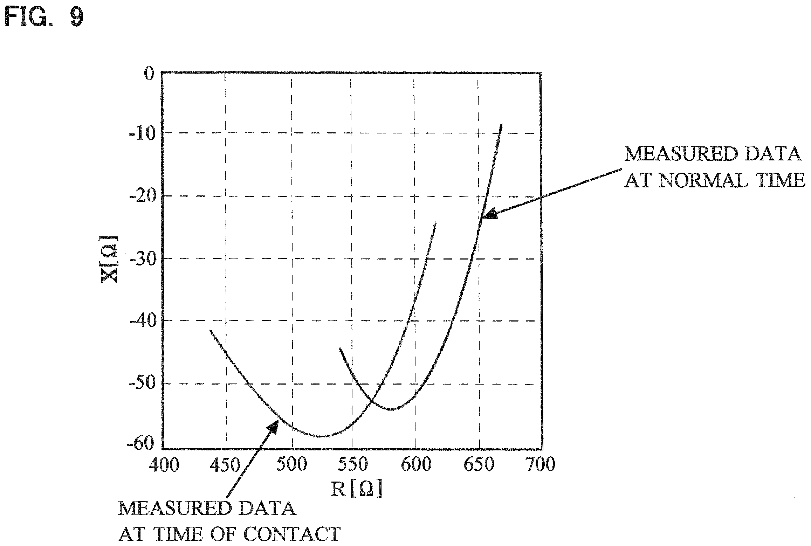

Next, referring to FIG. 8 and FIG. 9, it will be described how the measured values of the resistance R and the reactance X, when the bioelectrical impedance is measured at the time of contact between parts of the body of a subject with each other (hereafter sometimes referred to simply as "at the time of contact"), change compared with the normal time (when the bioelectrical impedance is measured in the normal posture). FIG. 8 shows result of plotting, on a complex plane, the measured values of the resistance R and the reactance X of a right arm collected by applying currents of the respective frequencies at the normal time and at the time of contact. In FIG. 8, the resistances R and the reactances X are shown on the horizontal axis (x-axis) and the vertical axis (y-axis), respectively. Further, the curved arrow ARW1 extending from low frequency to high frequency in this Figure indicates that the frequency of the current used to collect the measured data increases from right to left in this Figure.

At the normal time, as described in FIG. 6 above, a human body forms a series connection of circuits one of which is shown in FIG. 5. However, at the time of contact described above (when parts of the body of a subject contact with each other), as shown in FIG. 7, an electrical equivalent circuitry corresponding to tissue of the connected part (the resistance $R_s$ of skin and the electrical equivalent circuitry EC2 of tissue immediately below the skin) is connected in parallel to this series circuit. Thus, because of the nature of the calculation equation of the combined resistance value $R_p$ of resistances R connected in parallel ($1/R_p = 1/R_1 + 1/R_2$) (generally the combined resistance value $R_p$ of resistances R connected in parallel is lower than $R_1$ and $R_2$), the resistance of the electrical equivalent circuitry EC3 (after the

resistance $R_s$ of skin and the electrical equivalent circuitry EC2 of tissue immediately below the skin are connected in parallel) in FIG. 7 is lower than the resistance of the electrical equivalent circuitry EC1 of the left upper arm (before the resistance $R_s$ and the electrical equivalent circuitry EC2 are connected in parallel). Therefore, the center of the Cole circle moves in the negative direction of the x-axis. More specifically, as shown in FIG. 8, the center $O_3$ of the Cole circle of the measured data at the time of contact (Cole circle calculated using the resistances R and the reactances X of the right arm at the time of contact) moves in the negative direction of the x-axis (left direction in FIG. 8) from the center $O_0$ of the Cole circle of the measured data at the normal time (Cole circle calculated using the resistances R and the reactances X of the right arm at the normal time). In FIG. 8, assume that $v(v_x,v_y)$ is the vector starting at the center $O_0$ of the Cole circle of the measured data at the normal time and ending at the center $O_3$ of the Cole circle of the measured data at the time of contact. Note that the "Cole circle of measured data at normal time" in FIG. 8 corresponds to the "Cole circle of normal data" in the claims and FIG. 2.

FIG. 9 shows a locus (impedance locus) of the Cole-Cole plot at (measurement) points as generated based on the measured data (measured values of the resistance R and the reactance X at the time of applying current at the respective frequencies) of the right arm at the normal time and the measured data of the right arm at the time of contact in contact with the right side.

As described above, the center $O_3$ of the Cole circle of the measured data at the time of contact moves in the negative direction of the x-axis (left direction in FIG. 8) from the center $O_0$ of the Cole circle of the measured data (normal data) at the normal time. Therefore, the normal data Cole circle calculation circuitry 13 calculates a Cole circle of the normal data based on the data of normal bioelectrical impedances Z' collected from a group of many normal subjects and stored in the normal database, or on the data of past normal bioelectrical impedances Z' (intra-individual normal data) of the subject to be measured currently. Further, the measured data Cole circle calculation circuitry 12 calculates a Cole circle of the measured data by using the resistances R and the reactances X of the bioelectrical impedances Z at the respective frequencies (of a part to be measured) which are measured by the impedance measurement circuitry 11 described above.

After the calculation of each Cole circle described above is performed, the measurement anomaly determination circuitry 14 checks whether or not the vector $v(v_x,v_y)$ starting at the center of the Cole circle of the normal data and ending at the center of the Cole circle of the measured data satisfies both conditions ($V_x<0$) and ($|V_x|>\sigma$) (whether or not the center of the Cole circle of the measured data is shifted in a certain amount $\sigma$ or more in a direction to reduce the resistance R as seen from the center of the Cole circle of the normal data), thereby making it possible to detect a contact between parts of the body of the subject with each other. In other words, the measurement anomaly determination circuitry 14 determines the presence or absence of an anomaly in the measurement of bioelectrical impedance due to a contact between parts of the body of the subject with each other based on the direction, and the distance ($|V_x|$) in the direction of the x-axis, of the center of the Cole circle of the measured data as calculated by the measured data Cole circle calculation circuitry 12 relative to the center of the Cole circle of the normal data. Here, $\sigma$ described above is a threshold representing a normal range of $|V_x|$ (distance in the direction of the x-axis between the center of the Cole circle of the normal data and the center of the Cole circle of the measured data) calculated from the normal data stored in the normal database described above.

<2-3. Determination of anomaly in measurement of impedance due to cause (2)>

Further, concerning the anomaly in the measurement due to (2) above which is a contact failure between the body of a subject and hand/foot electrodes, it is possible to detect an anomaly in the measurement due to this cause by using a method similar to the device described in Patent Document 1. More specifically, it is considered that if the contact impedance (contact resistance) is higher than a certain threshold, the hand/foot of the subject does not firmly contact with the hand/foot electrodes. Thus, when the contact impedance measurement circuitry 18 measures a contact impedance with a magnitude higher than the threshold, the measurement anomaly determination circuitry 14 detects that an anomaly in the measurement due to a contact failure between the body of the subject and the hand/foot electrodes has occurred. In other words, the measurement anomaly determination circuitry 14 determines the presence or absence of an anomaly in the measurement of bioelectrical impedance due to a contact failure between the body of the subject and the measurement electrodes (hand/foot electrodes) based on the contact impedance measured by the contact impedance measurement circuitry 18.

When measuring contact impedance, the (measurement anomaly determination circuitry 14 of the) CPU 10 uses the electrode switching circuitry 6 to switch the electrodes to be connected to the multi-frequency current application circuitry 7 and the voltage measurement circuitry 8 in order to measure a contact impedance between the body of the subject and the electrodes used for the current measurement of bioelectrical impedance. More specifically, first, the (measurement anomaly determination circuitry 14 of the) CPU 10 uses the electrode switching circuitry 6 to perform the switching of electrodes to allow two current application electrodes used for the current measurement of bioelectrical impedance to be connected to the multi-frequency current application circuitry 7 and the voltage measurement circuitry 8. Then, the (measurement anomaly determination circuitry 14 of the) CPU 10 uses the contact impedance measurement circuitry 18 to measure the contact impedance along this current path. Subsequently, the (measurement anomaly determination circuitry 14 of the) CPU 10 uses the electrode switching circuitry 6 to perform the switching of electrodes to allow two voltage measurement electrodes used for the current measurement of bioelectrical impedance to be connected to the multi-frequency current application circuitry 7 and the voltage measurement circuitry 8. Then, the (measurement anomaly determination circuitry 14 of the) CPU 10 uses the contact impedance measurement circuitry 18 to measure the contact impedance along this voltage measurement path. The measurement anomaly determination circuitry 14 detects that an anomaly in the measurement due to a contact failure between the body of the subject and the hand/foot electrodes has occurred, when either the contact impedance measured along the path (current path) between the current application electrodes, or the contact impedance measured along the path (voltage measurement path) between the voltage measurement electrodes has a magnitude higher than the threshold.

For example, if the current application electrodes and the voltage measurement electrodes, which are used for the current measurement of bioelectrical impedance, are electrodes 2a and 5a and electrodes 2b and 5b, respectively, the (measurement anomaly determination circuitry 14 of the) CPU 10 first uses the electrode switching circuitry 6 to allow the current application electrodes 2a, 5a to be connected to the multi-frequency current application circuitry 7 and the voltage measurement circuitry 8, and uses the contact impedance measurement circuitry 18 to measure the contact impedance along this current path. The (measurement anomaly determination circuitry 14 of the) CPU 10 subsequently uses the electrode switching circuitry 6 to allow the voltage measurement electrodes 2b, 5b to be connected to the multi-frequency current application circuitry 7 and the voltage measurement circuitry 8, and uses the contact impedance measurement circuitry 18 to measure the contact impedance along this voltage measurement path. Then, the measurement anomaly determination circuitry 14 detects that an anomaly in the measurement due to a contact failure between the body of the subject and the hand/foot electrodes has occurred, when either the contact impedance measured along the current path between the current application electrodes 2a, 5a, or the contact impedance measured along the voltage measurement path between the voltage measurement electrodes 2b, 5b has a magnitude higher than the threshold.

<2-4. Determination of anomaly in measurement of impedance due to cause (3)>

Further, an anomaly in the measurement due to (3) above (extreme dryness of the hand or foot of a subject) can be detected by using time series data of contact impedance. This time series data of contact impedance is generated as follows. More specifically, the time series data of contact impedance is generated such that the (measurement anomaly determination circuitry 14 of the) CPU 10 uses the contact impedance measurement circuitry 18 to measure the contact impedance at a given sampling period based on the measurement method described above, and successively stores the measured contact impedances in the RAM 21 or the flash ROM 20. Based on this time series data of contact impedance measured by the contact impedance measurement circuitry 18, the measurement anomaly determination circuitry 14 determines the presence or absence of an anomaly in the measurement of bioelectrical impedance due to the dryness of the hand or foot of the subject.

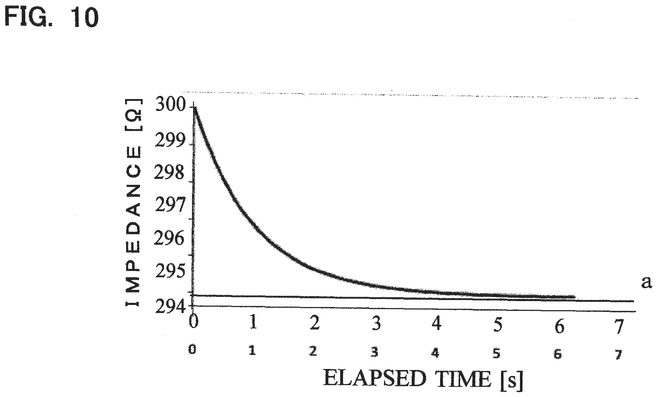

The detection of an anomaly in the measurement due to the dryness of the hand/foot of the subject using the time series data of contact impedance is performed as follows. Generally, when the hand/foot of a subject is dry, there is a tendency that the contact impedance gradually decreases as time elapses. An example is that $\Delta Z$ (= $Z_{current\ value}$ - $Z_{previous\ value}$) is continuously negative for 3 seconds. More specifically, this means that if it is assumed that the contact impedance is sampled every one second, then (contact impedance $Z_0$ at the time of the first measurement) > (contact impedance $Z_1$ 1 second after the first measurement) > (contact impedance $Z_2$ 2 seconds after the first measurement) > (contact impedance $Z_3$ 3 seconds after the first measurement). This is because when the hand/foot contacts with the measurement electrode (hand/foot electrode), the intimacy of contact between the measurement electrode and the hand/foot increases due to sweat and so on as time elapses. More specifically, as shown in FIG. 10, the contact impedance gradually decreases until it reaches a certain convergence value a(Q), and approaches the convergence value a. Therefore, by capturing the variation in the time derivative value of the contact impedance, an anomaly in the measurement due to the dryness of the hand or foot of the subject can be detected.

Thus, the measurement anomaly determination circuitry 14 determines, based on the time series data of the contact impedance described above, whether or not there is a tendency that the contact impedance gradually decreases as time elapses, and determines that an anomaly in the measurement of bioelectrical impedance due to the dryness of the hand or foot of the subject is occurring when it determines that there is the tendency of decrease. Note that it is also possible that the measurement anomaly determination circuitry 14 determines, based on the time series data of the contact impedance described above, whether or not the degree of decrease in the contact impedance exceeds a threshold, and determines that an anomaly in the measurement of bioelectrical impedance due to the dryness of the hand or foot of the subject is occurring when it determines that the threshold is exceeded.

<3. Operation of body composition meter>

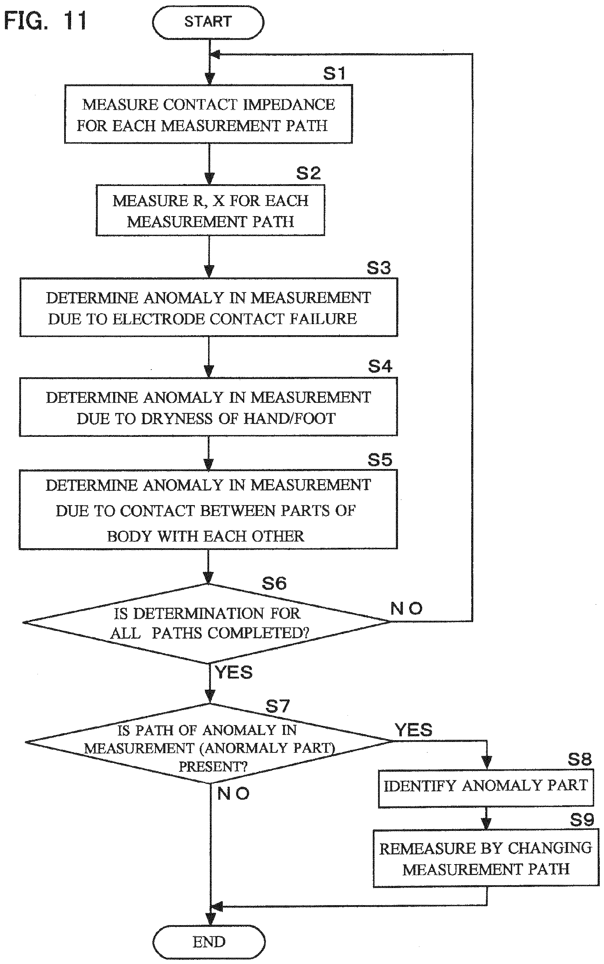

FIG. 11 is a flow chart showing processes for determination of an anomaly in the measurement of bioelectrical impedance in the process performed by the present body composition meter 1. In the processes shown in FIG. 11, the CPU 10 of the body composition meter 1 executes a process of identifying a cause of an anomaly in the measurement of bioelectrical impedance, a process of identifying a part (anomaly part) of the body of a subject acting as a source of producing an anomaly in the measurement, and a process of remeasurement (to measure again) by changing the measurement path when an anomaly part is present.

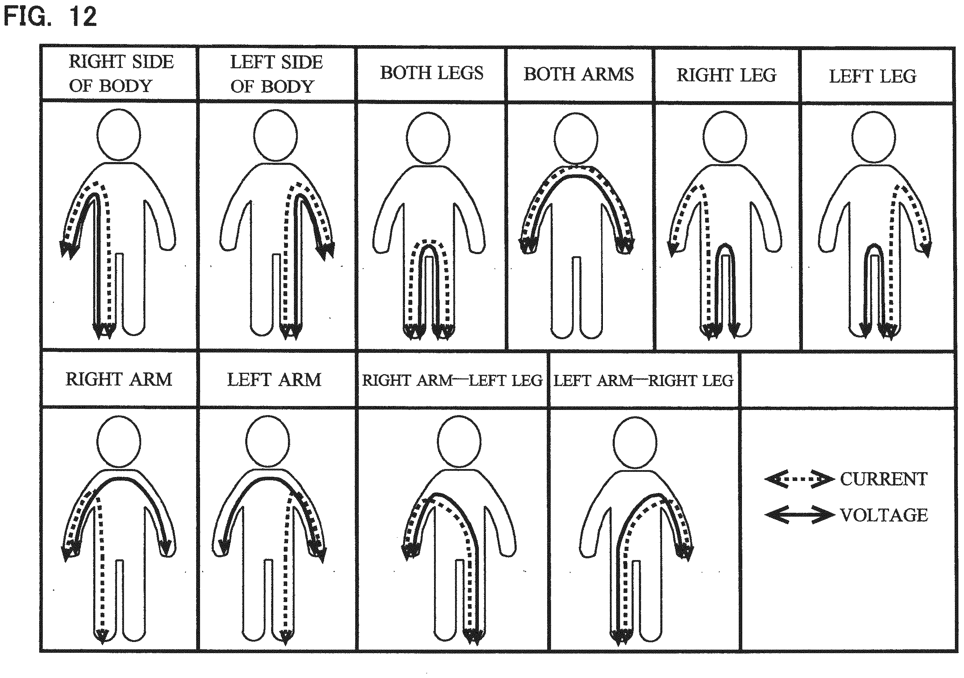

Specifically, the (measurement anomaly determination circuitry 14 of the) CPU 10 of the body composition meter 1 selects one of the 10 measurement paths shown in FIG. 12, and uses the contact impedance measurement circuitry 18 to measure the contact impedances along the current path and the voltage measurement path included in the selected measurement path (S1). Further, the (measurement anomaly determination circuitry 14 of the) CPU 10 of the body composition meter 1 repeats the measurement of contact impedance described above at a given sampling period so as to generate time series data of the contact impedance described above.

Next, the (measurement anomaly determination circuitry 14 of the) CPU 10 of the body composition meter 1 uses the impedance measurement circuitry 11 to calculate (measure) the resistances R and the reactances X at the respective frequencies along the selected measurement path (S2). Then, based on the contact impedances measured in S1 above, the (measurement anomaly determination circuitry 14 of the) CPU 10 of the body composition meter 1 determines the presence or absence of an anomaly in the measurement of bioelectrical impedance due to a contact failure between the body of the subject and the measurement electrodes (hand/foot electrodes) (S3).

Next, based on the time series data of contact impedance generated from the contact impedances measured in S1 above, the (measurement anomaly determination circuitry 14 of the) CPU 10 determines the presence or absence of an anomaly in the measurement of bioelectrical impedance due to the dryness of the hand or foot of the subject (S4).

Then, the (measurement anomaly determination circuitry 14, mainly, of the) CPU 10 uses the measured data Cole circle calculation circuitry 12 to calculate a Cole circle of measured data using the resistances R and the reactances X measured at the respective frequencies in S2 above, and determines the presence or absence of an anomaly in the measurement of bioelectrical impedance due to a contact between parts of the body of the subject with each other based on an amount of shift of the Cole circle of the measured data relative to the Cole circle of the normal data (S5). Note that the determination of the presence or absence of an anomaly in the measurement due to the contact between the parts of the body with each other is preferably performed based on whether or not the center of the Cole circle of the measured data is shifted in a certain amount $\sigma$ or more in a direction to reduce the resistance R as seen from the center of the Cole circle of the normal data.

If the (measurement anomaly determination circuitry 14 of the) CPU 10 determines the presence of an anomaly in the measurement in at least one of the determinations in S3 to S5 above, it determines that there is some anomaly in measurement in the measurement path. The (measurement anomaly determination circuitry 14 of the) CPU 10 executes the determinations of S3 to S5 above for all the 10 measurement paths shown in FIG. 12. Then, if a measurement path with an anomaly (in measurement) is present (an anomaly part is present) (YES in S7) as a result of the determinations of anomaly in measurement along all the measurement paths (YES in S6), the (anomaly part identification circuitry 16 of the) CPU 10 identifies a part (an anomaly part) of the body of the subject acting as a source of producing the anomaly in the measurement based on the results of the determinations of anomaly in measurement in the plurality of measurement paths (S8). Further, if an anomaly in the measurement is present in one of the plurality of measurement paths, the (remeasurement control circuitry 17 of the) CPU 10 controls the impedance measurement circuitry 11 to remeasure the bioelectrical impedance of the part to be measured along this measurement path by using a measurement path different from the measurement path which is normally used for the impedance measurement of this part (by changing the measurement path) (S9).

Next, referring to FIG. 12, the identification process of an anomaly part indicated in S8 above will be described in detail. In FIG. 12, the dashed line curve with arrows represents a current path, while the solid line curve with arrows represents a voltage measurement path. Based on combined results of the determinations of anomaly in measurement in a plurality of measurement paths among the 10 measurement paths shown in FIG. 12, the anomaly part identification circuitry 16 identifies where, among the right arm, left arm, right leg and left leg of the subject, the anomaly is occurring. The following describes the reason why the anomaly part identification circuitry 16 uses combined results of the determinations of anomaly in measurement in a plurality of measurement paths to identify the part of the subject where the anomaly is occurring.

A premise is that from only the result of determination of an anomaly in the measurement of impedance along one measurement path, it is not possible to identify which part of the subject included in the one measurement path is a cause of the anomaly in the measurement of impedance. For example, when an anomaly in the measurement of impedance occurs in the measurement path of the right side of the body shown in FIG. 12, it is not possible to identify, from only the result of determination of an anomaly in the measurement of impedance in this measurement path, whether the anomaly part is the right arm or the right leg.

Further, the case where parts of the body of the subject contact with each other gives an influence on the bioelectrical impedance along a measurement path which has a voltage measurement path including a voltage measurement electrode corresponding to one part in contact with the other part. However, this case does not give an influence on the bioelectrical impedance along a measurement path which does not have a voltage measurement path including a voltage measurement electrode corresponding to the one part in contact with the other part, and which has a current measurement path including a current measurement electrode corresponding to the one part in contact with the other part. For example, the case where the right arm contacts with the body trunk gives an influence on the bioelectrical impedance along the measurement paths which have a voltage measurement path including the voltage measurement electrode 3b, that is, along all of the measurement path of the right side of the body, the measurement path of both arms, the measurement path of the right arm, the measurement path of the left arm, and the measurement path between the right arm and the left leg as shown in FIG. 12.

Furthermore, the case where there is a contact failure between the body of the subject and the hand/foot electrodes and the case where the hand or foot of the subject is extremely dry give an influence on the contact impedance along a measurement path which includes a voltage measurement electrode or a current measurement electrode causing the contact failure with the body of the subject, and a measurement path which includes a voltage measurement electrode or a current measurement electrode in contact with the extremely dry hand or foot of the subject. For example, the case where there is a contact failure between the right hand and the electrodes 3a, 3b of the right hand gives an influence on the contact impedance along the measurement paths which have a current application path including the current application electrode 3a and the measurement paths which have a voltage measurement path including the voltage measurement electrode 3b, that is, along all of the measurement path of the right side of the body, the measurement path of both arms, the measurement path of the right leg, the measurement path of the right arm, the measurement path of the left arm, and the measurement path between the right arm and the left leg as shown in FIG. 12.

For the reasons described above, the anomaly part identification circuitry 16 uses combined results of the determinations of anomaly in measurement in a plurality of measurement paths to identify a part of the subject where the anomaly is occurring. The results of the identification of the anomaly part described above can be informed to the subject by a method such as displaying on the display unit 19, and can also be used to determine the measurement path to be used for the remeasurement in the remeasurement process indicated in S9 above.

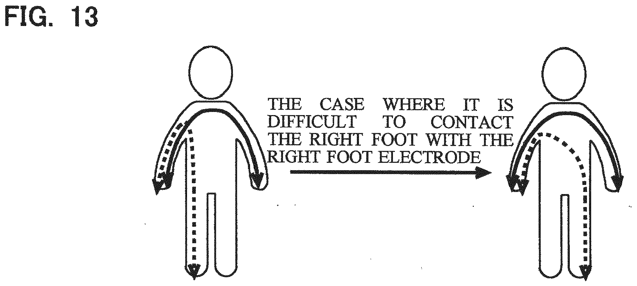

Next, referring to FIG. 13, the remeasurement process indicated in S9 above will be described in detail. In FIG. 13, the dashed line curve with arrows represents a current path, while the solid line curve with arrows represents a voltage measurement path. If a measurement path with an anomaly (in measurement) is present (an anomaly part is present) as a result of the determination indicated in S7 above, the (remeasurement control circuitry 17 of the) CPU 10 switches the current path without changing the voltage measurement path in the measurement paths so as to make it possible to measure (collect) accurate values of bioelectrical impedance instead of values of bioelectrical impedance at the time of the anomaly in the measurement. This makes it possible that the bioelectrical impedance of a part to be measured along the original measurement path can be measured (collected) by changing the measurement path of the bioelectrical impedance even if, for example, a hand or foot on one side is lost, and even if it is difficult to contact the four limbs with the electrodes due to the presence of paralysis.

For example, if it is difficult to contact the right foot with the current application electrode for right foot (corresponding to 5a in FIG. 1), the (remeasurement control circuitry 17 of the) CPU 10 can change (switch) the current path shown by the dashed line as shown in FIG. 13, and use the impedance measurement circuitry 11 to measure the bioelectrical impedance of the right arm (part to be measured along the original measurement path) by using a current path after the change. In FIG. 13, the current path is switched from the current path, which connects the right leg and the right arm (current path for current to flow through from the right leg to the right arm), to the current path which connects the left leg and the right arm (current path for current to flow through from the left leg to the right arm). Note that when determining the measurement path to be used for this remeasurement, the (remeasurement control circuitry 17 of the) CPU 10 can use the processing result of the identification process of an anomaly part indicated in S8 above.

As described above, the body composition meter 1 of the present exemplary embodiment is configured to determine the presence or absence of an anomaly in the measurement of bioelectrical impedance based on an amount of shift of a Cole circle of measured data relative to a Cole circle of normal data calculated using data of normal bioelectrical impedance. Thus, in contrast to the device described in Patent Document 2 above, the presence or absence of an anomaly in the measurement of bioelectrical impedance can be accurately determined by using the Cole circle of normal data as a reference, even if it is difficult to determine the anomaly in the measurement of bioelectrical impedance based on the magnitude of variation of measured values of the bioelectrical impedance of a subject at the respective frequencies relative to an impedance locus, as in the case where parts of the body of the subject contact with each other. Therefore, it is possible to accurately detect an anomaly in the measurement of bioelectrical impedance due to a contact between parts of the body of the subject with each other. Thus, even if there occurs an anomaly in the measurement of bioelectrical impedance due to a contact between parts of the body of the subject with each other, the body composition meter 1 of the present exemplary embodiment makes it possible that by using a method such as informing the subject of this anomaly in measurement, the subject can be prevented from misunderstanding parameters on the body composition obtained based on inaccurate data of bioelectrical impedance as correct, and taking a wrong action such as excessive eating or exercise.

Further, the body composition meter 1 of the present exemplary embodiment is configured so that the measured data Cole circle calculation circuitry 12 uses the impedance measurement circuitry 11 to measure the resistances and reactances at three or more frequencies, or at two or more frequencies including at least a frequency of at least 20 kHz and at most 100 kHz, and uses the measured resistances and reactances to calculate a Cole circle of the measured data. Thus, as described in FIGs. 3(a), 3(b) above, the Cole circle can be reliably calculated (drawn).

Further, the body composition meter 1 of the present exemplary embodiment is configured so that the measurement anomaly determination circuitry 14 determines the presence or absence of an anomaly in the measurement of bioelectrical impedance due to a contact between parts of the body of the subject with each other based on a direction and a distance in the direction of the x-axis of the center of the Cole circle of the measured data as calculated by the measured data Cole circle calculation circuitry 12 relative to the center of the Cole circle of the normal data. Here, when parts of the body of the subject contact with each other, the center of the Cole circle of the measured data moves in the negative direction of the x-axis. Therefore, the presence or absence of an anomaly in the measurement of bioelectrical impedance due to a contact between parts of the body of the subject with each other can be accurately determined based on the direction and the distance in the direction of the x-axis of the center of the Cole circle of the measured data relative to the center of the Cole circle of the normal data.

Further, the body composition meter 1 of the present exemplary embodiment further comprises the contact impedance measure circuitry 18 configured to measure a contact impedance generated between a measurement electrode and the body of the subject, and is configured so that the measurement anomaly determination circuitry 14 performs not only a process to determine the presence or absence of an anomaly in the measurement of bioelectrical impedance due to a contact between parts of the body of the subject with each other based on the direction and the distance in the direction of the x-axis of the center of the Cole circle of the measured data relative to the center of the Cole circle of the normal data, but also a process to determine the presence or absence of an anomaly in the measurement of bioelectrical impedance due to a contact failure between a measurement electrode and the body of the subject based on the contact impedance measured by the contact impedance measurement circuitry 18, and a process to determine the presence or absence of an anomaly in the measurement of bioelectrical impedance due to dryness of the hand or foot of the subject based on time series data of the contact impedance measured by the contact impedance measurement circuitry 18. Thus, it is possible to identify which of the contact between parts of the body of the subject with each other, the contact failure between the measurement electrode and the body of the subject, and the dryness of the hand or foot of the subject is a cause of the anomaly in the measurement of bioelectrical impedance.

Further, the body composition meter 1 of the present exemplary embodiment is configured to comprise the remeasurement control circuitry 17 which is configured so that if an anomaly in measurement is present in one of a plurality of measurement paths, the remeasurement control circuitry 17 controls the impedance measurement circuitry 11 to remeasure the bioelectrical impedance of the part to be measured along this measurement path by using a measurement path different from the measurement path which is normally used for the impedance measurement of this part. This makes it possible that the bioelectrical impedance of a part to be measured along the original measurement path can be measured even if, for example, a hand or foot on one side is lost, and even if it is difficult to contact the four limbs with the electrodes due to the presence of paralysis.

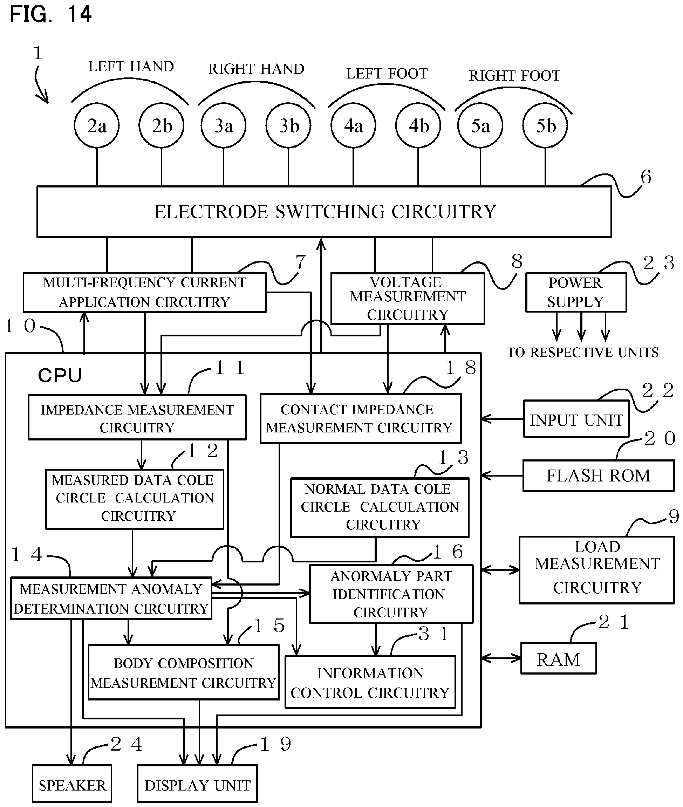

Next, referring to FIG. 14, a body composition meter 1 according to a second exemplary embodiment of the present invention will be described. The electrical block diagram of the body composition meter 1 of the second exemplary embodiment shown in FIG. 14 is similar to that of the body composition meter 1 of the first exemplary embodiment, except that it does not comprise the remeasurement control circuitry 17 (refer to FIG. 1) in the first exemplary embodiment, and comprises an information control circuitry 31. Thus, the structural elements (blocks) in FIG. 14 other than the information control circuitry 31 are given the same reference numerals as those of the respective blocks in FIG. 1, and their description is omitted. The information control circuitry 31 corresponds to the information control unit in the claims.

When the measurement anomaly determination circuitry 14 determines the presence of an anomaly in the measurement of bioelectrical impedance, the information control circuitry 31 controls to provide information to urge the subject to take appropriate countermeasures (such as taking an appropriate posture) depending on the cause of the anomaly in the measurement. For example, if the part to be measured is the left arm (refer to FIG. 12) when the measurement anomaly determination circuitry 14 determines the presence of an anomaly in the measurement of bioelectrical impedance due to a contact between parts of the body of the subject with each other, the information control circuitry 31 displays the contact area (in this case, between the left upper limb and the body trunk) on the display unit 19, and also displays (presents), on the display unit 19, a message to urge to prevent the left upper limb from contacting with the body trunk by providing a space between the left upper limb and the body trunk, or by placing an electrically insulating material (such as towel) between the left upper limb and the body trunk. Further, when the measurement anomaly determination circuitry 14 determines the presence of an anomaly in the measurement of bioelectrical impedance due to dryness of the hand or foot of the subject, a message such as "the hand/foot is dry and therefore should be made wet with water" is displayed (presented) on the display unit 19. Note that instead of the display unit 19, a speaker 24 can also be used for the above-described information (such as presentation of a message) to urge the subject to take appropriate countermeasures. Further, the information control circuitry 31 can use the result of the anomaly part identification process indicated in S8 in FIG. 11 above to change the details of the countermeasures to be taken by the subject.

Note here that the bioimpedance measurement device described in Patent Document 1 above and the body composition estimation device described in Patent Document 2 above can detect that an anomaly in the measurement of bioelectrical impedance is occurring due to some cause (undesirable measurement situation). However, they cannot identify the cause of the anomaly in the measurement of bioelectrical impedance, and therefore cannot provide the above-described information to urge a subject to take appropriate countermeasures. Thus, these devices cannot show how to improve the undesirable measurement situation for the subject (what countermeasures to take), and therefore have not made it possible to obtain accurate values of bioelectrical impedance of the subject. In contrast, as described above, the body composition meter 1 of the present exemplary embodiment makes it possible to identify the cause of the anomaly in the measurement of bioelectrical impedance, and provide information to urge the subject to take appropriate countermeasures. Therefore, based on this information, the subject can take appropriate countermeasures. Thus, the body composition meter 1 of the present exemplary embodiment can obtain accurate values of bioelectrical impedance of the subject, therefore making it possible to accurately measure the body composition based on the accurate values of bioelectrical impedance.

[Modified Examples]

It is to be noted that the present invention is not limited to the exemplary embodiments described above, and various modifications are possible within the spirit and scope of the present invention. Modified examples of the present invention will be described below.

[Modified Example 1]

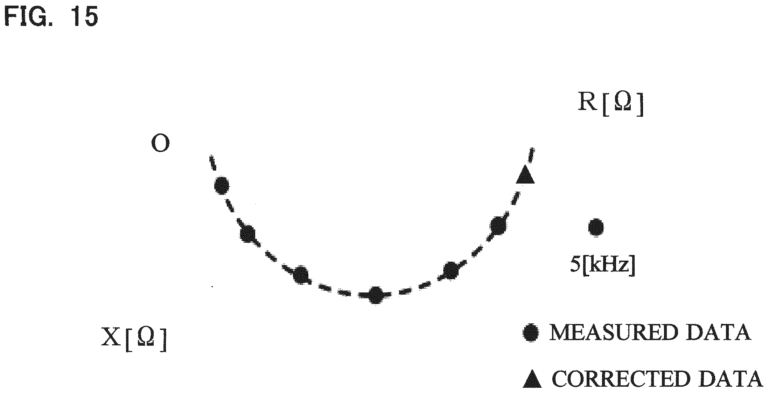

According to the first exemplary embodiment described above, if an anomaly in measurement is present in one of a plurality of measurement paths, the CPU 10 remeasures the bioelectrical impedance of a part to be measured along this measurement path by changing the measurement path. However, even if an anomaly in measurement is present in one of the plurality of measurement paths, it is also possible that if the bioelectrical impedance can be correctly measured along this measurement path at three or more frequencies, the bioelectrical impedances (resistances and reactances) measured at these three or more frequencies can be used to estimate (obtain) an appropriate Cole circle, and correct the value (measured data) of the bioelectrical impedance measured at a frequency when correct measurement could not be performed (an anomaly in the measurement was present). In this case, the CPU 10 determines whether or not correct measurement could be performed, based on whether it falls out of the normal range of impedance at each frequency as shown in FIG. 4 (normal range calculated from the group normal data, or normal range calculated from the intra-individual normal data). As shown in FIG. 15, a specific correction method is such that if the measured data at 5 [kHz], for example, is abnormal (falls out of the normal range of impedance at 5 [kHz]), data which could be correctly measured at other than 5 [kHz] (which does not fall out of the normal range of impedance) is used to estimate a Cole circle and correct the measured data at 5 [kHz]. More specifically, the measured data at 5 [kHz] is replaced by the value of bioelectrical impedance (resistance and reactance) (the corrected data in FIG. 15) at a point on the Cole circle which corresponds to the frequency of 5 [kHz].

[Modified Example 2]

Further, it is also possible to store the results of "determine anomaly in measurement due to contact of parts of body with each other" (refer to S5 of FIG. 11) of each subject for a given period in the flash ROM 20, and perform a character analysis on each subject based on the data of the results of determinations of anomaly in measurement for the given period. Specifically, a high likelihood of contact between parts of the body of a subject with each other (mainly, contact between an upper limb and the body trunk of the subject, or contact between the left and right lower limbs of the subject with each other) indicates that the subject takes an irregular posture during measurement, and the irregular posture during measurement indicates that the subject has a tendency of laziness. Therefore, based on the ratio of the data, in which an anomaly in measurement due to a contact between parts of the body with each other has been determined to be present, to the data of the results of the determinations of anomaly in measurement for the given period, the character analysis on the subject (analysis of whether the subject has a tendency of laziness) can be performed.

[Modified Example 3]

Further, based on the results of the determinations of "anomaly in measurement due to contact of parts of body with each other" as described above and the BMI (Body Mass Index) of each subject, the body shape of the subject can also be estimated (detected). For example, when the BMI value of the subject indicates that the subject has a slender shape, and if a contact between the left and right lower limbs of the subject with each other is detected, the subject is considered to have a skeleton which is likely to cause the thighs to contact. Thus, in this case, the CPU 10 detects that the subject has knock-kneed legs. Here, the CPU 10 detects the contact between the left and right lower limbs with each other as follows. More specifically, for example, when the measurement anomaly determination circuitry 14 detects the presence of an anomaly in the measurement of bioelectrical impedance due to a contact between parts of the body of the subject with each other, and if the parts to be measured are both legs (refer to FIG. 12), the CPU 10 detects a contact between the left and right lower limbs of the subject with each other. It is also possible for the CPU 10 to propose, to the subject, clothes fitted to the body shape detected (estimated) as above.

[Modified Example 4]

Further, if the body composition meter 1 outputs similar results of determinations of anomaly in the measurement (of bioelectrical impedance) for any subject, the anomaly in the measurement is highly likely to be one which is due to an anomaly in the device itself of the body composition meter 1, not an anomaly in the measurement due to the body of the subject such as a contact between parts of the body of the subject with each other, a contact failure between the body of the subject and measurement electrodes, and dryness of the hand or foot of the subject. Therefore, in this case, the CPU 10 can control to present, to a user such as the subject, that the device itself of the body composition meter 1 is highly likely to be in failure. More specifically, for example, a message such as "the body composition meter is highly likely to be in failure" is displayed on the display unit 19.

[Modified Example 5]

The respective exemplary embodiments described above have shown examples in which the normal data Cole circle calculation circuitry 13 in the CPU 10 of the body composition meter 1 calculates a Cole circle of normal data, and the calculated Cole circle of normal data is used to determine the presence or absence of an anomaly in the measurement of bioelectrical impedance. However, the body composition meter of the present invention is not limited to this. For example, it can be configured so that a Cole circle generated (calculated) from group normal data is stored in a flash ROM upon shipment of the body composition meter, and this Cole circle of normal data is used to determine the presence or absence of an anomaly in the measurement of bioelectrical impedance.

[Modified Example 6]

Further, the body composition measurement circuitry can be configured to obtain parameters on the body composition of a subject based on the bioelectrical impedance measured by the impedance measurement circuitry, only when the measurement anomaly determination circuitry determines the absence of an anomaly in the measurement of bioelectrical impedance, or to obtain parameters on the body composition of the subject not only when the measurement anomaly determination circuitry determines the absence of an anomaly in the measurement of bioelectrical impedance, but also when it determines the presence of an anomaly in the measurement.

[Modified Example 7]

The respective exemplary embodiments described above have described examples in which the body composition 1 is a body composition meter using 8 electrodes (a body composition meter of a type which measures both hands and feet). However, the body composition meter of the present invention is not limited to this, and can be a body composition meter using 4 electrodes (a body composition meter of a type which measures both feet).

[Modified Example 8]

The body composition meter 1 according to the second exemplary embodiment of the present invention described above does not comprise the remeasurement control circuitry 17 (refer to FIG. 1). However, as a modified example of the body composition meter 1 of the second exemplary embodiment, it is possible to allow it to further comprise the remeasurement control circuitry 17. More specifically, it can be configured so that when the measurement anomaly determination circuitry 14 determines the presence of an anomaly in the measurement of bioelectrical impedance based on the result of remeasurement by the remeasurement control circuitry 17, the information control circuitry 31 controls to provide information to urge the subject to take appropriate countermeasures (such as taking an appropriate posture) depending on the cause of the anomaly in the measurement.

[Modified Example 9]

The respective exemplary embodiments described above do not necessarily comprise the anomaly part identification circuitry 16. In this case, it can be configured so that when the measurement anomaly determination circuitry 14 determines the presence of an anomaly part (YES in S7 in the flow chart of FIG. 11), the information control circuitry 31 controls to provide information indicating the presence of an anomaly part.

[Modified Example 10]

In the respective exemplary embodiments described above, the CPU 10 of the body composition meter 1 comprises the impedance measurement circuitry 11, the measured data Cole circle calculation circuitry 12, the normal data Cole circle calculation circuitry 13, the measurement anomaly determination circuitry 14, the body composition measurement circuitry 15, the anomaly part identification circuitry 16, the remeasurement control circuitry 17, the contact impedance measurement circuitry 18 and the information control circuitry 31. However, it can also be configured that a processing unit of a computer which is separate from the body composition meter 1, and which comprises at least a processing unit such as CPU and a storage unit such as flash ROM and RAM, functions as at least one of the impedance measurement circuitry 11, the measured data Cole circle calculation circuitry 12, the normal data Cole circle calculation circuitry 13, the measurement anomaly determination circuitry 14, the body composition measurement circuitry 15, the anomaly part identification circuitry 16, the remeasurement control circuitry 17, the contact impedance measurement circuitry 18 and the information control circuitry 31. In this case, the storage unit of such computer or a storage media as a storage device to be connected to such computer stores a program (body composition measurement program) to allow the processing unit of such computer to function as each of the circuitries. More specifically, such computer executes such program to receive output current values of AC current and detected voltage values from a general body composition meter connected thereto via wired or wireless, and executes the steps for the determination of an anomaly in the measurement of impedance as described above in the respective exemplary embodiments. Note that examples of such computer are a PC (Personal Computer), a mobile terminal such as a smartphone, and so on.

## DESCRIPTION OF THE REFERENCE NUMERALS

| 1 | Body composition meter |
|---|---|
| 7 | Multi-frequency current application circuitry (multi-frequency current application unit) |
| 11 | Impedance measurement circuitry (impedance measurement unit) |
| 12 | Measured data Cole circle calculation circuitry (measured data Cole circle calculation unit) |
| 14 | Measurement anomaly determination circuitry (measurement anomaly determination unit) |
| 15 | Body composition measurement circuitry (body composition measurement unit) |
| 16 | Anomaly part identification circuitry (anomaly part identification unit) |
| 17 | Remeasurement control circuitry (remeasurement control unit) |
| 18 | Contact impedance measurement circuitry (contact impedance measurement unit) |
| 31 | Information control circuitry (information control unit) |
| C0 | Cole circle of normal data |
| C1 | Cole circle of measured data |
| $O_0$ | Center of Cole circle of normal data |
| $O_1, O_3$ | Center of Cole circle of measured data |
| AR1 | Area excluded from within Cole circle of normal data |
| r0 | Radius of Cole circle of normal data |
| r2 | Radius of Cole circle of measured data |
| $|Vx|$ | Distance in x-axis direction |

## Claims

1. A body composition meter comprising:

a multi-frequency current application unit configured to apply an AC current of multiple frequencies to a body of a subject;

an impedance measurement unit configured to measure bioelectrical impedances of the subject when the AC current is applied by the multi-frequency current application unit at the respective frequencies;

a body composition measurement unit configured to obtain parameters on a body composition of the subject based on the bioelectrical impedances measured by the impedance measurement unit;

a measured data Cole circle calculation unit configured to calculate a Cole circle of measured data using resistances and reactances in the bioelectrical impedances at the respective frequencies as measured by the impedance measurement unit; and

a measurement anomaly determination unit configured to determine the presence or absence of an anomaly in the measurement of the bioelectrical impedance based on an amount of shift of the Cole circle of the measured data calculated by the measured data Cole circle calculation unit relative to a Cole circle of normal data using normal data of bioelectrical impedance.

2. The body composition meter according to claim 1,
wherein the measured data Cole circle calculation unit calculates the Cole circle of the measured data by using the resistances and reactances measured by the impedance measurement unit at three or more frequencies, or at two or more frequencies including at least a frequency of at least 20 kHz and at most 100 kHz.

3. The body composition meter according to claim 1 or 2,
wherein the measurement anomaly determination unit determines the present or absence of an anomaly in the measurement of the bioelectrical impedance based on an amount of shift of the center of the Cole circle of the measured data calculated by the measured data Cole circle calculation unit relative to the center of the Cole circle of the normal data.

4. The body composition meter according to claim 1 or 2,
wherein the measurement anomaly determination unit determines the presence of absence of an anomaly in the measurement of the bioelectrical impedance based on an area which is a region within the Cole circle of the measured data as calculated by the measured data Cole circle calculation unit, and which is excluded from within the Cole circle of the normal data.

5. The body composition meter according to claim 1 or 2,
wherein the measurement anomaly determination unit determines the presence of absence of an anomaly in the measurement of the bioelectrical impedance based on an amount of difference between a radius of the Cole circle of the normal data and a radius of the Cole circle of the measured data as calculated by the measured data Cole circle calculation unit.

6. The body composition meter according to any of claims 1 to 5,
wherein the measurement anomaly determination unit determines the presence of absence of an anomaly in the measurement of the bioelectrical impedance due to a contact between parts of the body of the subject with each other based on a direction and a distance in an x-axis direction of the center of the Cole circle of the measured data as calculated by the measured data Cole circle calculation unit relative to the center of the Cole circle of the normal data.

7. The body composition meter according to claim 6, further comprising a contact impedance measurement unit configured to measure a contact impedance generated between a measurement electrode used to measure the bioelectrical impedance of the subject and the body of the subject,
wherein the measurement anomaly determination unit performs not only a process to determine the presence of an anomaly in the measurement of bioelectrical impedance due to the contact between the parts of the body of the subject with each other based on the direction and the distance in the direction of the x-axis of the center of the Cole circle of the measured data relative to the center of the Cole circle of the normal data, but also a process to determine the presence or absence of an anomaly in the measurement of bioelectrical impedance due to a contact failure between the measurement electrode and the body of the subject based on the contact impedance measured by the contact impedance measurement unit, and a process to determine the presence or absence of an anomaly in the measurement of bioelectrical impedance due to dryness of a hand or foot of the subject based on time series data of the contact impedance measured by the contact impedance measurement unit.

8. The body composition meter according to any of claims 1 to 7, further comprising an anomaly part identification unit configured so that when the measurement anomaly determination unit determines the presence or absence of an anomaly in the measurement of the bioelectrical impedance along a plurality of measurement paths of the body of the subject, and if the anomaly in the measurement is present in one of the plurality of measurement paths, the anomaly part identification unit identifies a part of the body of the subject acting as a source of producing an anomaly in measurement based on results of the determinations of anomaly in measurement in the plurality of measurement paths.

9. The body composition meter according to claim 8, further comprising a remeasurement control unit configured so that if the anomaly in measurement is present in one of the plurality of measurement paths, the remeasurement control unit controls the impedance measurement unit to remeasure the bioelectrical impedance of the part to be measured along this measurement path by using a measurement path different from the measurement path which is normally used for the impedance measurement of this part.

10. The body composition meter according to claim 7 or 8, further comprising an information control unit configured so that when the measurement anomaly determination unit determines the presence of an anomaly in the measurement of bioelectrical impedance, the information control unit controls to provide information to urge the subject to take appropriate countermeasures depending on the cause of the anomaly in the measurement.

11. A body composition measurement program to cause a computer to execute the steps of:

measuring bioelectrical impedances of a subject when an AC current of multiple frequencies is applied to a body of the subject at the respective frequencies;
obtaining parameters on a body composition of the subject based on the bioelectrical impedances measured by the step of measuring the bioelectrical impedance of the subject;
calculating a Cole circle of measured data using resistances and reactances in the bioelectrical impedances at the respective frequencies as measured by the step of measuring the bioelectrical impedance of the subject; and
determining the presence or absence of an anomaly in the measurement of the bioelectrical impedance based on an amount of shift of the Cole circle of the measured data calculated by the step of calculating the Cole circle of the measured data relative to a Cole circle of normal data using normal data of bioelectrical impedance.

## FIG. 1

1

LEFT HAND    RIGHT HAND    LEFT FOOT    RIGHT FOOT

2a  2b    3a  3b    4a  4b    5a  5b

6

ELECTRODE SWITCHING CIRCUITRY

7

MULTI-FREQUENCY CURRENT
APPLICATION CIRCUITRY

8

VOLTAGE
MEASUREMENT
CIRCUITRY

23

POWER
SUPPLY

TO RESPECTIVE UNITS

10

CPU

11

IMPEDANCE MEASUREMENT
CIRCUITRY

18

CONTACT IMPEDANCE
MEASUREMENT CIRCUITRY

22

INPUT UNIT

12

MEASURED DATA COLE
CIRCLE  CALCULATION
CIRCUITRY

13

NORMAL DATA COLE
CIRCLE  CALCULATION
CIRCUITRY

20

FLASH ROM

9

LOAD
MEASUREMENT
CIRCUITRY

14

MEASUREMENT ANOMALY
DETERMINATION CIRCUITRY

16

ANORMALY PART
IDENTIFICATION
CIRCUITRY

15

BODY COMPOSITION
MEASUREMENT CIRCUITRY

17

REMEASUREMENT
CONTROL CIRCUITRY

21

RAM

24

SPEAKER

19

DISPLAY UNIT

FIG. 2

Legend:
- ★ CENTER $O_0$ OF COLE CIRCLE C0 OF NORMAL DATA
- — COLE CIRCLE C0 OF NORMAL DATA
- ○ MEASUREMENT POINT OF MEASURED DATA 1 AT EACH FREQUENCY
- ---- COLE CIRCLE C1 OF MEASURED DATA 1
- ☆ CENTER $O_1$ OF COLE CIRCLE C1 OF MEASURED DATA 1
- ● MEASUREMENT POINT OF MEASURED DATA 2 AT EACH FREQUENCY
- —·— COLE CIRCLE C2 OF MEASURED DATA 2
- ✳ CENTER $O_2$ OF COLE CIRCLE C2 OF MEASURED DATA 2
- —·· NORMAL RANGE LMT OF CENTER OF CIRCLE

EP 3 708 076 A1

# FIG. 3

( a )

( b )

FIG. 4

NORMAL DATA OF CERTAIN SUBJECT

NORMAL RANGE CALCULATED FROM INTRA-INDIVIDUAL NORMAL DATA

NORMAL RANGE CALCULATED FROM GROUP NORMAL DATA

ABNORMAL DATA OF CERTAIN SUBJECT

X[Ω]

R[Ω]

## FIG.5

EXTRACELLULAR FLUID

$R_e$

$C_m$

$R_i$

INTRACELLULAR FLUID

CELL MEMBRANE

## FIG.6

RIGHT UPPER ARM

LEFT UPPER ARM

RIGHT LOWER ARM

LEFT LOWER ARM

FIG.7

EQUIVALENT CIRCUITRY EC2 OF
TISSUE IMMEDIATELY BELOW SKIN

RESISTANCE Rs OF SKIN

## FIG. 8

EP 3 708 076 A1

- ● MEASURED DATA AT NORMAL TIME
- · MEASURED DATA AT TIME OF CONTACT
- ★ CENTER O₀ OF COLE CIRCLE OF MEASURED DATA AT NORMAL TIME
- ☆ CENTER O₃ OF COLE CIRCLE OF MEASURED DATA AT TIME OF CONTACT

FIG. 9

EP 3 708 076 A1

FIG. 10

FIG. 11

```
                    ┌─────────┐
                    │  START  │
                    └────┬────┘
                         │←─────────────────────────────┐
                         ▼                               │
               ┌──────────────────────────────┐ S1      │
               │  MEASURE CONTACT IMPEDANCE    │         │
               │  FOR EACH MEASUREMENT PATH    │         │
               └──────────────┬───────────────┘         │
                              ▼                          │
               ┌──────────────────────────────┐ S2      │
               │  MEASURE R, X FOR EACH        │         │
               │  MEASUREMENT PATH             │         │
               └──────────────┬───────────────┘         │
                              ▼                          │
               ┌──────────────────────────────┐ S3      │
               │  DETERMINE ANOMALY IN         │         │
               │  MEASUREMENT DUE TO ELECTRODE │         │
               │  CONTACT FAILURE              │         │
               └──────────────┬───────────────┘         │
                              ▼                          │
               ┌──────────────────────────────┐ S4      │
               │  DETERMINE ANOMALY IN         │         │
               │  MEASUREMENT DUE TO DRYNESS   │         │
               │  OF HAND/FOOT                 │         │
               └──────────────┬───────────────┘         │
                              ▼                          │
               ┌──────────────────────────────┐ S5      │
               │  DETERMINE ANOMALY IN         │         │
               │  MEASUREMENT DUE TO CONTACT   │         │
               │  BETWEEN PARTS OF BODY        │         │
               │  WITH EACH OTHER              │         │
               └──────────────┬───────────────┘         │
                              ▼                   S6     │
                         ◇─────────────◇    NO           │
                        ╱ IS DETERMINATION╲──────────────┘
                        ╲ FOR ALL PATHS    ╱
                         ◇ COMPLETED?     ◇
                              │ YES
                              ▼                   S7
                         ◇─────────────◇    YES
                        ╱ IS PATH OF      ╲───────────┐
                        ╲ ANOMALY IN       ╱          ▼
                         ◇ MEASUREMENT    ◇    ┌──────────────┐ S8
                         (ANOMALY PART)        │ IDENTIFY     │
                          PRESENT?             │ ANOMALY PART │
                              │ NO             └──────┬───────┘
                              │                       ▼
                              │                ┌──────────────────┐ S9
                              │                │ REMEASURE BY     │
                              │                │ CHANGING         │
                              │                │ MEASUREMENT PATH │
                              │                └──────┬───────────┘
                              ▼←─────────────────────┘
                         ┌─────────┐
                         │   END   │
                         └─────────┘
```

## FIG. 12

| RIGHT SIDE OF BODY | LEFT SIDE OF BODY | BOTH LEGS | BOTH ARMS | RIGHT LEG | LEFT LEG |
|---|---|---|---|---|---|
| | | | | | |

| RIGHT ARM | LEFT ARM | RIGHT ARM—LEFT LEG | LEFT ARM—RIGHT LEG | |
|---|---|---|---|---|
| | | | | ◄----► CURRENT<br>◄——► VOLTAGE |

EP 3 708 076 A1

FIG. 13

THE CASE WHERE IT IS DIFFICULT TO CONTACT THE RIGHT FOOT WITH THE RIGHT FOOT ELECTRODE

# FIG. 14

1

LEFT HAND   RIGHT HAND   LEFT FOOT   RIGHT FOOT

(2a) (2b)   (3a) (3b)   (4a) (4b)   (5a) (5b)

6

ELECTRODE SWITCHING CIRCUITRY

7                                    8        2 3

| MULTI-FREQUENCY CURRENT APPLICATION CIRCUITRY | VOLTAGE MEASUREMENT CIRCUITRY | POWER SUPPLY |

TO RESPECTIVE UNITS

1 0

CPU

1 1

| IMPEDANCE MEASUREMENT CIRCUITRY |

1 8

| CONTACT IMPEDANCE MEASUREMENT CIRCUITRY |

2 2

| INPUT UNIT |

1 2

| MEASURED DATA COLE CIRCLE CALCULATION CIRCUITRY |

1 3

| NORMAL DATA COLE CIRCLE CALCULATION CIRCUITRY |

2 0

| FLASH ROM |

9

| LOAD MEASUREMENT CIRCUITRY |

1 4

| MEASUREMENT ANOMALY DETERMINATION CIRCUITRY |

1 6

| ANORMALY PART IDENTIFICATION CIRCUITRY |

1 5

| BODY COMPOSITION MEASUREMENT CIRCUITRY |

3 1

| INFORMATION CONTROL CIRCUITRY |

2 1

| RAM |

2 4

| SPEAKER |

1 9

| DISPLAY UNIT |

FIG. 15

O

R[Ω]

X[Ω]

5[kHz]

● MEASURED DATA

▲ CORRECTED DATA

## INTERNATIONAL SEARCH REPORT

| International application No. |
| --- |
| PCT/JP2018/039292 |

| A. CLASSIFICATION OF SUBJECT MATTER |
| --- |
| Int.Cl. A61B5/053(2006.01)i |

According to International Patent Classification (IPC) or to both national classification and IPC

| B. FIELDS SEARCHED |
| --- |

Minimum documentation searched (classification system followed by classification symbols)
Int.Cl. A61B5/053

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| X<br>A | JP 2009-112554 A (SEKISUI CHEMICAL CO., LTD.) 28 May 2009, paragraphs [0031], [0045]-[0053], [0068]-[0069], [0091], fig. 4-6 (Family: none) | 1, 2, 5, 11<br>3, 4, 6-10 |
| A | JP 2004-337308 A (SEKISUI CHEMICAL CO., LTD.) 02 December 2004, entire text, all drawings (Family: none) | 1-11 |
| A | JP 2002-10988 A (TANITA CORP.) 15 January 2002, entire text, all drawings & US 2001/0030546 A1, entire text, all drawings & EP 1138259 A2 & CN 1319376 A | 1-11 |

☒ Further documents are listed in the continuation of Box C.   ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 15 January 2019 (15.01.2019) | 29 January 2019 (29.01.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

| International application No. |
| --- |
| PCT/JP2018/039292 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | US 2013/0172776 A1 (IMPEDIMED LIMITED) 04 July 2013, entire text, all drawings & JP 2013-533031 A & WO 2012/000017 A1 & EP 2587997 A1 & CA 2802505 A1 | 1-11 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012213458 A

- US 5110277 A